# EUROPEAN PATENT SPECIFICATION

(11) **EP 2 760 832 B1**
(45) Date of publication and mention of the grant of the patent: **22.07.2015**
(21) Application number: 12766452.2
(22) Date of filing: 28.09.2012
(51) Int. Cl.: C07D 211/70, C07D 401/04, C07F 9/24

(54) **IMPROVED PROCESS FOR PREPARING 2-[(2E)-2-FLUORO-2-(3-PIPERIDINYLIDENE)ETHYL]-1H-ISOINDOLE-1,3(2H)-DIONE**
VERBESSERTES VERFAHREN ZUR HERSTELLUNG VON 2-[(2E)-2-FLUOR-2-(3-PIPERIDINYLIDEN)ETHYL]-1H-ISOINDOL-1,3(2H)-DION
PROCÉDÉ AMÉLIORÉ DE PRÉPARATION DE 2-[(2E)-2-FLUORO-2-(3-PIPERIDINYLIDENE)ETHYL]-1H-ISOINDOLE-1,3(2H)-DIONE

(30) Priority: 30.09.2011 EP 11183477
(43) Date of publication of application: 06.08.2014
(73) Proprietor: Janssen Pharmaceutica, N.V., 2340 Beerse (BE)
(72) Inventor: LANG, Yolande Lydia, B-2340 Beerse (BE); DEPRÉ, Dominique Paul Michel, B-2340 Beerse (BE)
(74) Representative: Verberckmoes, Filip Gerard
(86) International application number: PCT/EP2012/069164
(87) International publication number: WO 2013/045599

(56) References cited:
- WO-A1-2006/101603
- WO-A2-2008/005670
- WO-A2-2010/056633
- MORROW B.J. ET AL.: "In vitro antibacterial activities of JNJ-Q2, a new broad-spectrum fluoroquinolone", ANTIMICROBIAL AGENTS AND CHEMOTHERAPY, vol. 54, no. 5, 2010, pages 1955-1964, XP002667620, cited in the application

## Description

### Field of the invention

The present invention relates to an improved process for preparing 2-[(2*E*)-2-fluoro-2-(3-piperidinylidene)ethyl]-1*H*-isoindole-1,3(2*H*)-dione, or a salt thereof, which is an intermediate in the synthesis route of the antibacterial compound 7-[(3*E*)-3-(2-amino-1-fluoroethylidene)-1-piperidinyl]-1-cyclopropyl-6-fluoro-1,4-dihydro-8-methoxy-4-oxo 3-quinolinecarboxylic acid.

### Background of the Invention

WO-2006/101603 describes 7-amino alkylidenyl-heterocyclic quinolones as antimicrobial compounds and the synthesis of 7-[(3*E*)-3-(2-amino-1-fluoroethylidene)-1-piperidinyl]-1-cyclopropyl-6-fluoro-1,4-dihydro-8-methoxy-4-oxo 3-quinolinecarboxylic acid is disclosed as compound (303) in Table 1 on page 20. This compound is conveniently referred to as compound 'A' hereafter.

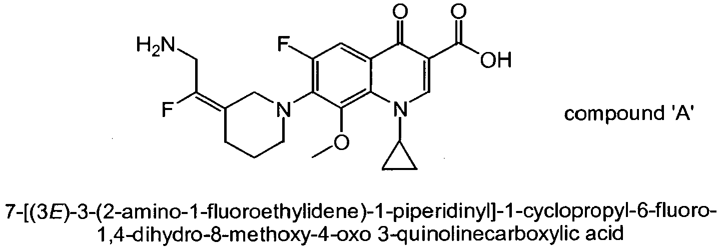

The *in vitro* antibacterial properties of compound 'A' are described by Morrow B.J. et al. in Antimicrobial Agents and Chemotherapy, vol. 54, pp. 1995 - 1964 (2010).

WO-2008/005670 discloses one-pot methods for the production of substituted allylic alcohols as well as extractive methods for the separation of certain isomeric alcohol products which are useful for preparing quinolones such as the antimicrobial compound 7-[(3*E*)-3-(2-amino-1-fluoroethylidene)-1-piperidinyl]-1-cyclopropyl-6-fluoro-1,4-dihydro-8-methoxy-4-oxo 3-quinolinecarboxylic acid (*i.e.* compound 'A').

An important intermediate in the overall synthesis route of said antimicrobial compound `A' is 2-[(2*E*)-2-fluoro-2-(3-piperidinylidene)ethyl]-1*H*-isoindole-1,3(2*H*)-dione and its hydrochloric acid salt thereof:

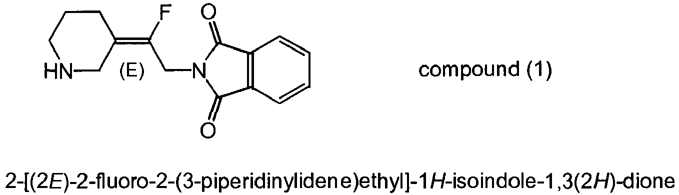

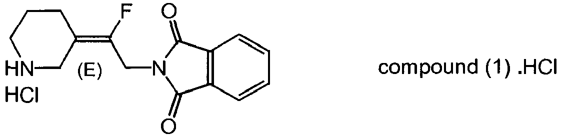

Compound (1) introduces the desired *E*-stereochemistry into the overall synthesis route for the antimicrobial compound `A'.

WO-2008/005670 discloses a synthesis route for compound (1) on page 38 as depicted below :

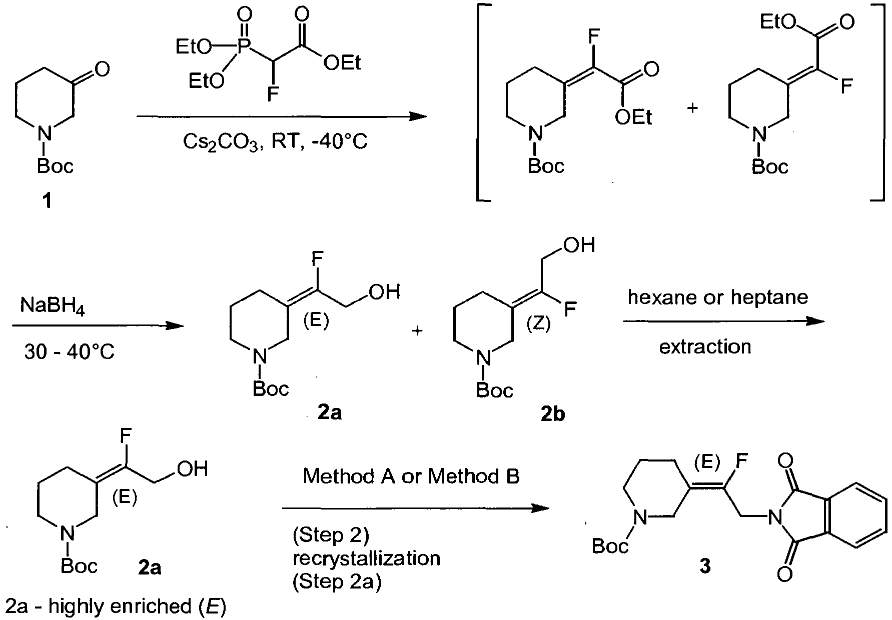

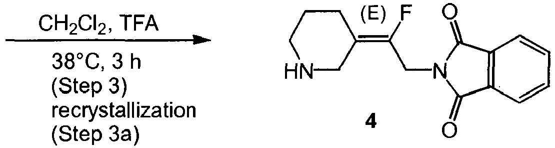

The detailed reaction procedure for compound (1) is disclosed in WO-2008/005670 in Example 1 on pages 37 to 44 affording compound (1) in Method A with a E:Z ratio of 97:3 in an approximate overall yield of 18 % in Method A (step 1 for the first 3 heptane layers has a yield of 34 % with a ratio E:Z of 71:29, step 2a has a yield of 53.4% with a ratio E:Z of 97:3, and step 3 has quantitave yield), or affording compound (1) in Method B with an approximate overall yield of 15% with a ratio E:Z of 94.4 : 5.6.

WO-2008/005670 discloses a synthesis route for the hydrochloric acid addition salt of compound (1) on page 15 in Scheme 2 as depicted below :

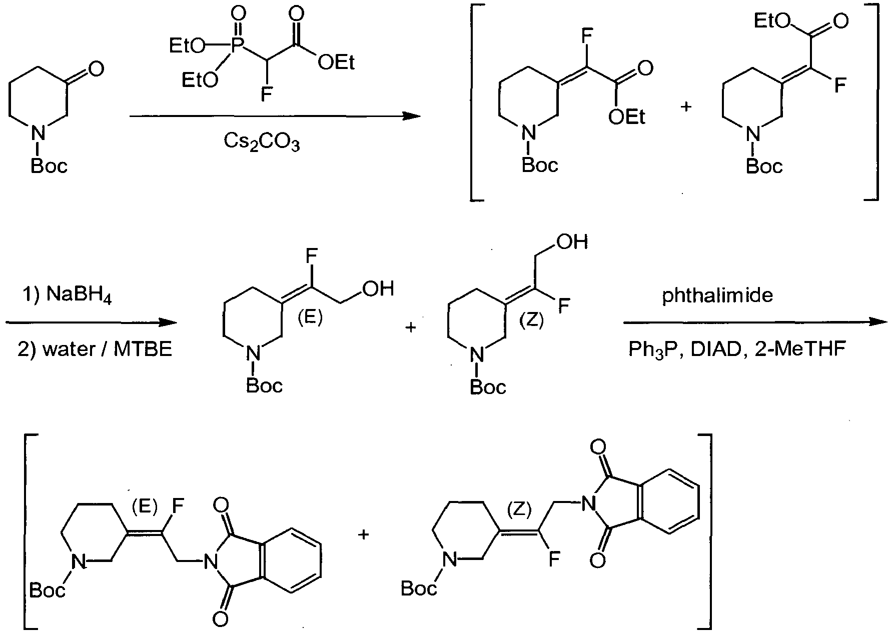

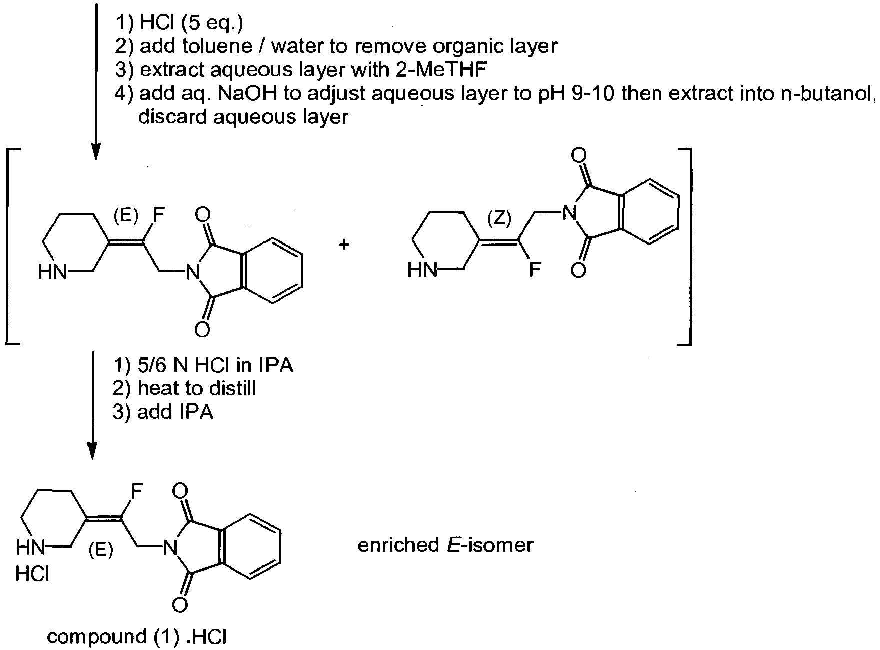

The detailed reaction procedure to prepare the HCl salt of compound (1) is disclosed in WO-2008/005670 in Example 4 on pages 49 to 52 affording >95% of desired *E*-isomer with an overall yield of 18 - 22% starting from N-boc-3-piperidone.

The reaction procedures described in WO-2008/005670 for the preparation of compound (1) or its HCl salt are characterized by lack of selectivity of the Wadsworth-Emmons-Homer reaction which produces the undesired Z-isomer in large quantities. This undesired Z-isomer requires additional time consuming separation steps.

Hence there is a need for a more efficient and less waste-producing procedure for the preparation of compound (1) or its HCl salt.

WO-2010/056633 discloses a synthesis scheme XIV on page 87 to prepare *tert*-butyl 4-(2-ethoxy-2-oxoethylidene)piperidinyl-1-carboxylate and a synthesis scheme XXVI on page 111 to prepare (1-benzyl-piperidin-4-ylidene)bromoacetic acid ethyl ester.

In a first embodiment the present invention relates to an improved process for preparing compounds of formula (III) having an improved ratio of the desired (*E*)-isomer over the undesired (Z)-isomer.

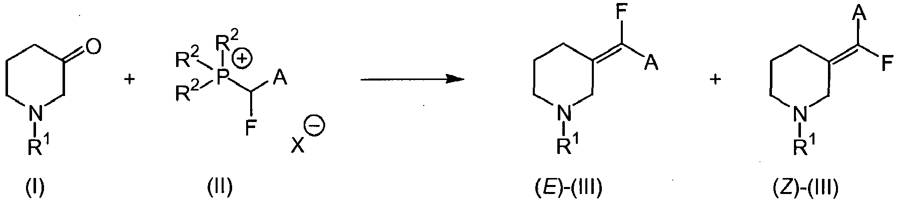

In a further embodiment the compound (*E*)-(III) is then converted in to compound (1) or its hydrochloric acid addition salt thereof.

### Description of the Invention

In one aspect, the present invention relates to a process for preparing a compound of formula (III), which is characterized by the steps of
reacting a compound of formula (I) with a compound of formula (II) in a reaction-inert solvent 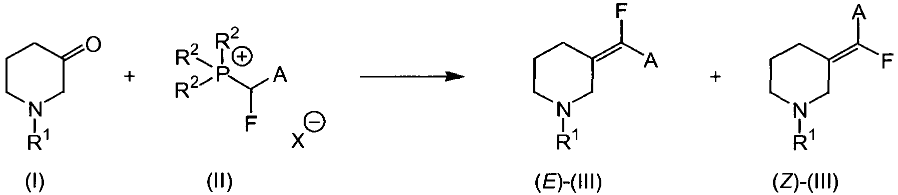 wherein
R¹ is hydrogen, C₁₋₄alkyl, aryl, arylmethyl, arylethyl, diphenylmethyl, allyl or 3-phenylallyl (cinnamyl);
R² is C₁₋₆alkyl, phenyl or phenyl substituted with one substituent selected from halo, hydroxy, C₁₋₄alkyl, C₁₋₄alkoxy, trifluoromethyl, cyano or nitro;
A is C₁₋₄alkyloxycarbonyl, hydroxycarbonyl, aminocarbonyl, or cyano; and
X is halo;
wherein aryl is phenyl, or phenyl substituted with one or two C₁₋₄alkyloxy, halo, C₁₋₄alkyl, nitro, or di(C₁₋₄alkyl)amino.

The reaction-inert solvent can be any solvent such as dichloromethane, acetonitrile, ethyl acetate, heptanes, tetrahydrofuran (THF), cyclopentyl methyl ether (CPME), di-n-butyl ether (DBE), methylcyclohexane (MeCy), chlorobenzene, fluorobenzene, N,N-dimethylacetamide, N,N-dimethylformadide, toluene, or anisole or any mixture thereof In practice the reaction-inert solvent is usually toluene.

In a second aspect, the present invention relates to a process of reducing compound (III), which is a mixture of (*E*)-(III) and (*Z*)-(III), into compound (IV), which is a mixture of (*E*)-(IV) and (*Z*)-(IV), followed by isolating compound (*E*)-(IV) or a salt thereof as a precipitate.

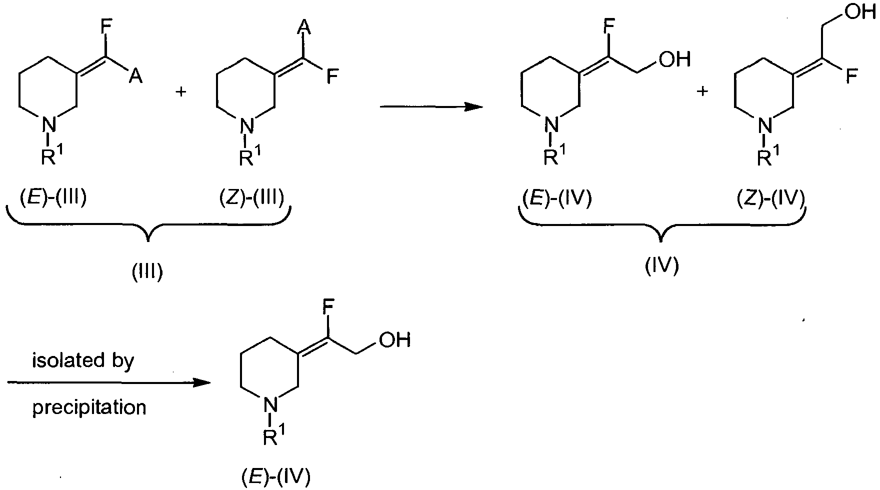

The reduction can be performed with any art-known reducing agent such as LiBH₄, LiAlH₄, sodium bis(2-methoxyethoxy) aluminumhydride (Red-Al), and silane reducing agents such as trialkylsilanes (e.g. trimethylsilane, triethylsilane, tris(trimethylsilyl)-silane), triphenylsilane, trialkoxysilanes (e.g. triethoxysilane) and polymeric siloxanes like poly(methylhydrosiloxane) in the presence of a catalyst.

The reduction reaction can be carried out in any suitable reaction-inert solvent such as e.g. toluene.

The precipitation of compounds (*E*)-(IV) is obtained by mixing compounds (IV) with a suitable solvent such as e.g. diisopropyl ether or di-n-butyl ether (DBE) followed by isolation of the precipitated compounds (*E*)-(IV). Optionally the solvent is warmed after addition of compounds (IV) and upon cooling the compounds (*E*)-(IV) precipitate.

In a third aspect, the present invention relates to a process for reacting a compound of formula (*E*)-(IV) with compound (V) under Mitsunobu reaction conditions thereby obtaining a compound of formula (VI) which can be converted into compound (1), optionally in the form of an addition salt, by removing substituent R¹ using art-known deprotection methods such as, e.g. hydrogenation or treatment with chloroformate.

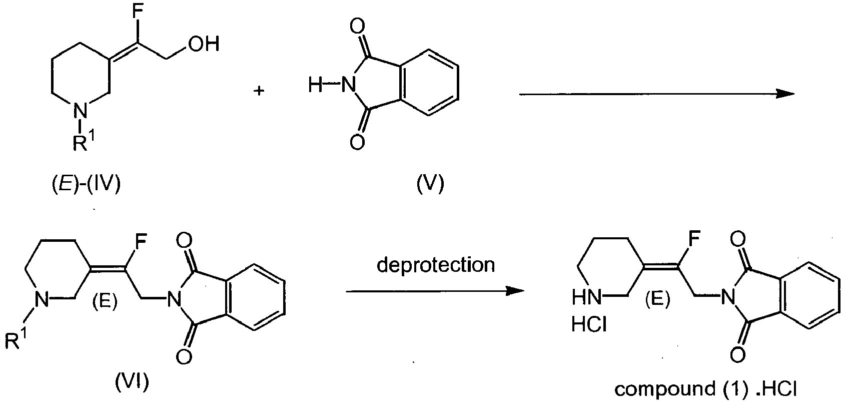

In a fourth aspect, the primary hydroxyl group in compound (*E*)-(IV) is first converted into a leaving group Y such as halo or a sulfonyloxy group, e.g. methanesulfonyloxy, benzenesulfonyloxy, trifluoromethanesulfonyloxy, para-toluenesulfonyloxy before conducting the reaction with compound (V) or a salt thereof (e.g. potassium salt) and removing substituent R¹ using the procedures as described above.

In a fifth aspect, the present invention also relates to novel compounds of formula (*E*)-(III) or (*E*)-(IV) 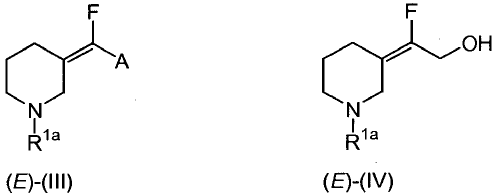 or acid addition salts thereof, wherein
R^{1a} is C₁₋₄alkyl, aryl, arylmethyl, arylethyl, diphenylmethyl, allyl or 3-phenylallyl (cinnamyl);
A is C₁₋₄alkyloxycarbonyl, hydroxycarbonyl, aminocarbonyl, or cyano;
wherein aryl is phenyl, or phenyl substituted with one or two C₁₋₄alkyloxy, halo, C₁₋₄alkyl, nitro, or di(C₁₋₄alkyl)amino.

As used in the foregoing definitions :
- halo is generic to fluoro, chloro, bromo and iodo;
- C₁₋₄alkyl defines straight and branched chain saturated hydrocarbon radicals having from 1 to 4 carbon atoms such as, for example, methyl, ethyl, propyl, butyl, 1-methylethyl, 2-methylpropyl and the like;
- C₁₋₆alkyl is meant to include C₁₋₄alkyl and the higher homologues thereof having 5 or 6 carbon atoms, such as, for example, 2-methylbutyl, pentyl, hexyl and the like.

The addition salts as mentioned hereinabove are meant to comprise the acid addition salt forms that the compounds of formula (I) are able to form. These acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (*i.e.* ethanedioic), malonic, succinic (*i.e.* butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids.

Conversely said salt forms can be converted by treatment with an appropriate base into the free base form.

Interesting processes of the present invention are those wherein one or more of the following restrictions apply :
a) R¹ is C₁₋₄alkyl, aryl, arylmethyl, arylethyl, diphenylmethyl, allyl or 3-phenylallyl; or
b) R¹ is methyl, ethyl, allyl, phenylethyl, diphenylmethyl, or arylmethyl wherein aryl is phenyl, or a methoxy substituted phenyl; in particular R¹ is arylmethyl wherein aryl is phenyl; or
c) R² is methyl, ethyl, butyl, isobutyl or phenyl; in particular R² is methyl; or
d) X is bromo; and
e) A is hydroxycarbonyl, aminocarbonyl, cyano or C₁₋₄alkyloxycarbonyl wherein C₁₋₄alkyl is methyl, ethyl, or *tert-*butyl; in particular A is C₁₋₄alkyloxycarbonyl wherein C₁₋₄alkyl is ethyl.

### Chemical definitions.

The term "isomer" means compounds that have the same composition and molecular weight but differ in physical and/or chemical properties. Such substances have the same number and kind of atoms but differ in structure. The structural difference may be in constitution (geometric isomers) or may result from different spatial arrangements of the groups in the molecule (stereoisomers).

The term "stereoisomer" means isomers of identical constitution that differ in the arrangement of their atoms in space. Enantiomers and diastereomers are stereoisomers wherein an asymmetrically substituted carbon atom acts as a chiral center. The term "chiral" refers to a molecule that is not superposable on its mirror image, implying the absence of an axis and a plane or center of symmetry.

An isomer is designated as being in the "Z" (zusammen = "together") configuration if the groups of highest priority lie on the same side of a reference plane passing through the double bond and perpendicular to the plane containing the bonds linking the groups to the double-bonded atoms; the other isomer is designated as "*E*" (entgegen = "opposite"). The term "priority" used to determine E and Z isomers herein refers to the rules established for the purpose of unambiguous designation of isomers described in R.S. Cahn, C.K. Ingold and V. Prelog.

### Experimental part

The following abbreviations will be used in the following part:

| | |
|---|---|
| AcOEt | ethyl acetate |
| MeTHF | 2-methyltetrahydrofuran |
| CPME | cyclopentyl methyl ether |
| DBE | di-n-butyl ether |
| DBU | 1,8-diazabicyclo [5.4.0]undec-7-ene |
| DCM | dichloromethane |
| DIAD | diisopropylazodicarboxylate |
| DIPE | diisopropyl ether |
| DMA | N,N-dimethylacetamide |
| DMF | N,N-dimethylformamide |
| MeCN | acetonitrile |
| MeCy | methylcyclohexane |
| MIBK | 5-methyl-2-pentanone |
| TBAI | tetrabutylammonium iodide |
| THF | tetrahydrofuran |
| Red-Al | sodium bis(2-methoxy-ethoxy)aluminumhydride |

### Example 1 : preparation of compounds of formula (I)

Compounds of formula (I) when isolated as a hydrochloric acid addition salt can be present either in the ketone form (I) or as in the gem-diol form (VII) ("hydrate") or as a mixture of both in a variable ratio. However, during the neutralization of the salt (formation of the free amine), the gem-diol spontaneously looses a molecule of water to form the ketone.

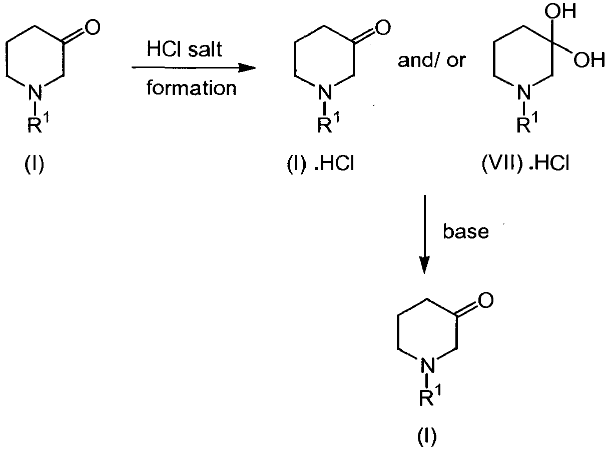

In the following text, the term "HCl salt of compound (I)" will always refer to either strictly speaking "HCl salt of compound (I)", to "HCl salt of compound (VII)" or to a mixture of both.

### Step 1 : synthesis of 3,3-dimethoxypiperidine

25 g (125.5 mmol) of N-boc-3-piperidone was dissolved in 125 ml of methanol. 9.1 ml (138 mmol) of methanesulfonic acid was added and the reaction mixture was stirred a few hours at room temperature then at 50°C until completion. The reaction mixture was neutralized with 15.96 g (150.6 mmol) of sodium carbonate, diluted with 125 ml of toluene, and the methanol was removed under vacuum. The resulting suspension was further diluted with 125 ml of toluene. The solid materials were filtered off and rinsed with 63 ml of toluene and the combined filtrates were concentrated under vacuum. The residue was distilled under reduced pressure (100-105°C, 25 mbar) to afford 7.61 g of 3,3-dimethoxypiperidine as a colorless liquid (yield : 42 %) (bp: 100-105°C, 25 mbar).
¹H NMR (360 MHz, chloroform-*d*) δ ppm 3.12 (s, 6 H), 2.71 (s, 2 H), 2.58 - 2.68 (m, 2 H), 1.63 - 1.70 (m, 2 H), 1.57 (br. s., 1 H), 1.43 - 1.51 (m, 2 H).
¹³C NMR (91 MHz, chloroform-*d*) δ ppm 96.69, 51.01, 47.31, 45.90, 31.05, 24.27. GC-MS (EI): 145 (M⁺), 130 (M - Me), 114 (M - MeO).

### Step 2 : synthesis of compound Ia) to (Ij)

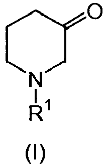

| Compound | R¹ |
|---|---|
| (Ia) .HCl | Bn |
| (Ib) .HCl | Me |
| (Ic) .HCl | Et |
| (Id) .HCl | Allyl |
| (Ie) .HCl | 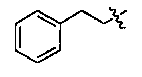 |
| (If) .HCl | 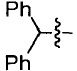 |
| (Ig) .HCl | H |
| (Ih).HCl | 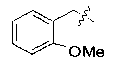 |
| (Ii) .HCl | 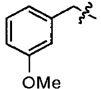 |
| (Ij) .HCl | 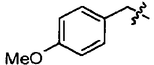 |

| | |
|---|---|
| Bn : benzyl Ph : phenyl Me : methyl Allyl: H₂C=CH-CH₂- Et : ethyl | |

### Compounds (Ia) .HCl salt and (Ic) .HCl salt are commercially available.

### Compounds (Ib) .HCl salt was prepared from 3-hydroxypyridine following a procedure of the literature (Lyle, R.E.; Adel, R.E., Lyle, G.G. J. Org. Chem. 1959, 24, 343).

### Synthesis of compound (Id) .HCl salt :

19.8 g (136 mmol) 3,3-dimethoxypiperidine and 19 ml (136 mmol) oftriethylamine were dissolved in 136 ml of THF. 15.6 ml (150 mmol) of allyl chloride was added and the reaction mixture was stirred at room temperature then at 50°C until complete conversion of the starting materials (2 days). After cooling to room temperature, the insoluble materials were filtered off and washed with a few ml of toluene.

The combined filtrate and washing layer were concentrated under vacuum to dryness. The oily residue was redissolved in 20 ml of ethyl acetate and filtered through a pad of silica gel. The pad was rinsed with 100 ml of ethyl acetate and the combined filtrates were concentrate under vacuum to deliver 20.5 g of N-allyl-3,3-dimethoxypiperidine whose structure was confirmed by NMR and LC-MS (yield : 81%). 19 g ofN-allyl-3,3-dimethoxypiperidine was dissolved in 100 ml of aqueous 3M HCl solution and 30 ml THF and stirred 4 hours at 60°C. Toluene was added to the reaction mixture and the layers were separated. The water layer was concentrated under vacuum to dryness and co-evaporated twice with dichloromethane. The resulting solid was stirred in a few ml of acetone then filtered and dried, yielding 8.5 g of compound (Id) HCl salt (yield : 47%).

### Compound (Id) .HCl salt (ratio ketone /(hydrate: about 1 /1) :

Appearance : white solid.
¹H NMR (360MHz ,DMSO-d₆, mixture of ketone and hydrate) δ = 12.19 (br. s., 0.55 H), 9.51 (br. s., 0.45 H), 6.10 - 5.85 (m, 1 H), 5.62 - 5.42 (m, 2 H), 3.92 (m, 0.55 H), 3.82 (d, *J* = 7.0 Hz, 1.1 H), 3.78 - 3.43 (m, 2.3 H), 3.39 - 3.04 (m, 1.4 H), 2.94 - 2.73 (m, 0.9 H), 2.67 - 2.38 (m, 0.5 H), 2.26 (br. s., 0.55 H), 2.12 (br. s., 0.55 H), 1.95 - 1.67 (m, 1.5 H), 1.62 - 1.44 (m, 0.55 H).
¹³C NMR (91 MHz, DMSO-*d*₆, signal for the ketone form) δ ppm 200.80, 126.93, 125.20, 58.49, 57.35, 48.69, 36.89, 19.17
¹³C NMR (91 MHz, DMSO-*d*₆, signal for the hydrate form) δ ppm 127.69, 124.78, 90.20, 58.41, 58.02, 51.35, 34.25, 19.72 High resolution MS: calculated for C₈H₁₄NO (ketone form, M + H): 140.1070, found: 140.1064. calculated for CₛH₁₆NO₂ (hydrate form, M + H): 158.1176, found: 158.1190.

### Synthesis of compound (Ie) HCl salt :

8.0 g (55.1 mmol) of 3,3-dimethoxypiperidine and 7.1 ml (60.6 mmol) of phenylacetaldehyde are dissolved in 83 ml of MeTHF and the solution is inerted. 2.37 g of wet 5% Pd/C are added and the reaction mixture is stirred for 5 hours at room temperature under 6 bar of hydrogen. After completion, the pressure is released, the catalyst is filtered off and the filtrate is concentrate under vacuum to dryness to afford 13.7 g of crude 3,3-dimethoxy-N-phenethylpiperidine (quantitative crude yield). The crude acetal is dissolved in 66 ml of aqueous 1M HCl. The aqueous solution is washed with 55 ml of isopropyl acetate then slowly concentrated under vacuum (temperature: 60°C) to dryness. 110 ml of MIBK were added and the resulting mixture was slowly evaporated under vacuum. 110 ml of MIBK were added again and the mixture was refluxed for 2 hours before being stirred overnight at room temperature. The suspension was concentrated to dryness and the residue was recrystallized from 30 ml of acetonitrile to yield 5.51 g of compound (Ie) .HCl salt (yield : 42%) as a pale yellow solid.

### Compound (Ie) : 3,3-dimethoxy-N-phenylethylpiperidine :

¹H NMR (360MHz, chloroform-d) δ = 7.30 - 7.23 (m, 2 H), 7.22 - 7.14 (m, 3 H), 3.22 (s, 6 H), 2.88 - 2.78 (m, 2 H), 2.67 - 2.56 (m, 2 H), 2.49 (d, *J* = 15.4 Hz, 4 H), 1.67 (br. s., 4 H).
¹³C NMR (91 MHz, chloroform-*d*) δ ppm 140.2, 128.5, 128.2, 125.8, 98.0, 60.5, 57.4, 53.7, 47.6, 33.2, 31.1, 22.2.
LC-MS: 21.38 (M - OMe), 250.34 (M + H).

### Compound (Ie) .HCl salt (mixture of ketone and hydrate):

¹H NMR (360 MHz, DMSO-*d*₆, signal for the ketone form) δ ppm 12.14 (br. s., 1 H), 7.33 - 7.39 (m, 2 H), 7.24 - 7.33 (m, 3 H), 3.99 (dd, *J*=15.4, 8.4 Hz, 1 H), 3.87 (d, *J*=15.4 Hz, 1 H), 3.73 (d, *J*=12.1 Hz, 1 H), 3.27 - 3.48 (m, 3 H), 3.07 - 3.18 (m, 2 H), 2.44 - 2.67 (m, 2 H), 2.24 - 2.42 (m, 1 H), 2.07 - 2.20 (m, 1 H)
¹³C NMR (151 MHz, DMSO-*d*₆, signals for the ketone form) δ ppm 200.90, 137.10, 128.76, 128.67, 126.84, 58.90, 56.66, 51.80, 34.42, 29.43, 19.86
¹³C NMR (151 MHz, DMSO-*d*₆, signals for the hydrate form) δ ppm 136.98, 128.73, 128.67, 126.84, 90.21, 59.14, 56.39, 49.50, 36.88, 29.19, 19.27
High resolution MS: calculated for C₁₃H₁₈NO (ketone form, M + H): 204.1388, found: 204.1395. calculated for C₁₃H₂₀NO₂ (hydrate form, M + H): 222.1489, found: 222.1551.

### Compounds (If) .HCl salt :

5.00 g (34 mmol) of 3,3-dimethoxypiperidine was dissolved in 69 ml of MeTHF. 5.71g (41 mmol) of potassium carbonate and 8.51 g (34 mmol) of bromodiphenylmethane were added and the reaction mixture was refluxed overnight. After cooling to room temperature, the inorganic materials were washed out with 34 ml of water. 39 ml of aqueous 1 M HCl solution were added to the organic layer and the resulting biphasic mixture was refluxed then concentrated under vacuum to dryness. The semi-solid residue was refluxed in 39 ml of MIBK. After cooling to room temperature, the solid was filtered, washed with a few of MIBK and dried, yielding 7.48 g of compound (If) .HCl salt as a tanned solid (yield: 72%).
¹H NMR (360 MHz, DMSO-*d*₆) δ ppm 13.12 (br. s., 1 H), 7.78 - 8.16 (m, 4 H), 7.20 - 7.56 (m, 6 H), 5.81 (d, *J*=7.7 Hz, 1 H), 3.85 (dd, *J*=13.2, 7.0 Hz, 1 H), 3.22 - 3.54 (m, 3 H), 2.55 (br. s., 2 H), 2.50 - 2.53 (m, 1 H), 2.12 (br. s., 1 H)
¹³C NMR (101 MHz, DMSO-*d*₆) δ ppm 199.93, 135.81, 134.96, 129.35, 129.22, 128.86, 128.56, 127.94, 73.96, 58.62, 49.56, 36.86, 18.54
High resolution MS: calculated for C₁₈H₂₀NO (ketone form, M + H): 266.1545, found: 266.1549. calculated for C₁₈H₂₂NO₂ (hydrate form, M + H): 284.1651, found: 284.1654.

### Compound (Ig) .HCl salt :

37.6 ml (37.6 mmol) of 1M aqueous HCl solution were added to 5 g (25.1 mmol) of N-boc-3-piperidone and the reaction mixture was stirred at room temperature for a few hours before concentration under vacuum. The residue was recrystallized from isopropanol to afford 3.40 g of compound (Ig). HCl salt as a solid (yield: 63 %).
¹H NMR (360 MHz, DMSO-d6) δ ppm 9.74 (br. s., 2 H), 3.66 (br. s., 2 H), 3.15 - 3.36 (m, 2 H), 2.50 (m,2H), 1.95 - 2.22 (m, 2 H)

### Compound (Ih) . HCl salt :

A mixture of 800 mg (5.51 mmol) of 3,3-dimethoxypiperidine and 750 mg (5.51 mmol) of 2-methoxybenzaldehyde in 8.3 ml of methanol was stirred under 1 bar of hydrogen in the presence of 59 mg (0.055 mmol) of 10w/w% Pd/C. After completion of the reaction (a few hours), the reaction mixture was purged with nitrogen, filtered and 6.6 ml of aqueous 1M HCl was added to the filtrate. The mixture was concentrated under vacuum to dryness. The residue was redissolved in 4.4 ml of hot acetone. 4.4 ml of MIBK was added to the hot solution, the mixture was refluxed for a few hours then cooled to room temperature. The suspension was filtered and the collected solid was washed with a few ml of MIBK then dried under vacuum to afford 950 mg of compound (Ih) .HCl salt as a off-white solid (yield : 68%).
Ratio ketone/ hydrate about 95/5.
¹H NMR (360 MHz, DMSO-*d*₆, signals for the ketone) δ ppm 11.88 (br. s., 1 H), 7.63 (dd, *J*=7.5, 1.6 Hz, 1 H), 7.35 - 7.55 (m, 1 H), 7.14 (d, *J*=7.7 Hz, 1 H), 7.03 (td, *J*=7.3, 0.7 Hz, 1 H), 4.35 (br. s., 2 H), 3.90 (dd, *J*=15.5, 9.5 Hz, 1 H), 3.86 (s, 3 H), 3.59 (d, *J*=15.0 Hz, 1 H), 3.50 (d, *J*=11.7 Hz, 1 H), 3.26 (q, *J*=9.9 Hz, 1 H), 2.25 - 2.62 (m, 3 H), 2.00 - 2.16 (m, 1 H).
¹³C NMR (91 MHz, DMSO-*d*₆, signals for the ketone form) δ ppm 200.64, 148.10, 133.37, 131.56, 120.47, 116.91, 111.47, 58.91, 55.69, 53.05, 48.90, 36.71, 19.25.
¹³C NMR (91 MHz, DMSO-*d*₆, signals for the hydrate form) δ ppm 148.10, 133.37, 131.56, 120.47, 116.91, 111.47, 90.18, 58.91, 55.69, 51.28, 41.06, 37.48, 19.93.
High resolution MS: calculated for C₁₃H₁₈NO₂ (ketone form, M + H) : 220.1332, found : 220.1326. calculated for C₁₃H₂₀NO₃ (hydrate form, M + H) : 238.1438, found: 238.1463.

### Compound (Ii) .HCl salt :

A mixture of 800 mg (5.51 mmol) of 3,3-dimethoxypiperidine and 750 mg (5.51 mmol) of 3-methoxybenzaldehyde in 8.3 ml of methanol was stirred under 1 bar of hydrogen in the presence of 59 mg (0.055 mmol) of 10w/w% Pd/C. After completion of the reaction (a few hours), the reaction mixture was purged with nitrogen, filtered and 6.6 ml of aqueous 1M HCl was added to the filtrate. The mixture was concentrated under vacuum to dryness. The residue was suspended in 4.4 ml of hot acetone. 4.4 ml of MIBK was added and acetone was distilled off. The suspension then cooled to room temperature and filtered. The collected solid was washed with a few ml of MIBK then dried under vacuum to afford 1.09 g of compound (Ii) .HCl salt as a off-white solid (yield: 77%).
High resolution MS : calculated for C₁₃H₁₈NO2 (ketone form, M + H) : 220.1332, found : 220.1312. calculated for C₁₃H₂₀NO₃ (hydrate form, M + H) : 238.1438, found : 238.1444.
Ratio ketone/hydrate : about 95/5.
¹H NMR (360 MHz, DMSO-*d*₆, signals for the ketone) δ ppm 12.20 (br. s., 1 H), 7.30 - 7.42 (m, 2 H), 7.17 (d, *J*=7.7 Hz, 1 H), 7.02 (dd, *J*=8.2, 2.4 Hz, 1 H), 4.38 (br. s., 2 H), 3.86 (dd, *J*= 15.0, 8.8 Hz, 1 H), 3.79 (s, 3 H), 3.57 (d, *J*= 15.0 Hz, 1 H), 3.50 (d, *J*=12.1 Hz, 1 H), 3.11 - 3.33 (m, 1 H), 2.4.1 - 2.63 (m, 2 H), 2.21 - 2.41 (m, 1 H), 2.10 (br. s., 1 H)
¹³C NMR (91 MHz, DMSO-*d*₆) δ ppm 200.55, 159.32, 130.70, 129.83, 123.30, 116.63, 115.26, 58.70, 58.39, 55.20, 48.88, 36.88, 19.09

### Example 2

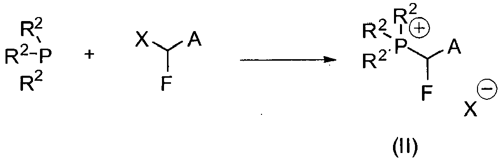

| Compound | R² | A | X | Yield |
|---|---|---|---|---|
| (IIa) | Me | CO₂Et | Br | 87% |
| (IIb) | Et | CO₂Et | Br | 84% |
| (IIc) | Bu | CO₂Et | Br | 100% (crude) |
| (IId) | iBu | CO₂Et | Br | 11% |
| (IIe) | Ph | CO₂Et | Br | 53% |
| (IIf) | Me | CO₂Me | Br | 95% |
| (IIg) | Me | CO₂tBu | Br | 73% |
| (IIh) | Me | CONH₂ | Br | 33% |
| (IIi) | Me | CO₂H | Br | |
| (IIj) | Me | CN | Br | |

### Compound (IIa) :

74 g (400 mmol) of ethyl bromofluoroacetate are added slowly to 400 ml (400 mmol) of 1M trimethylphosphine solution in toluene and the reaction mixture is stirred overnight at room temperature. The compound (IIa) is filtered, washed with 80 ml of toluene and dried under vacuum at 40°C. Yield: 90.97 g (87% yield).
Appearance : white solid.
¹H-NMR (360 MHz, CDCl₃) δ = 7.34 (dd, J = 7.0, 43.5 Hz, 1 H), 4.52 - 4.33 (m, 2 H), 2.49 (d, J = 15.0 Hz, 6 H), 1.39 (t, J = 7.1 Hz, 3 H).
¹³C-NMR (90.6 MHz, CDCl₃) δ =163.51 (dd, J = 1.8, 20.7 Hz), 83.80 (dd, J = 63.0, 206.3 Hz), 64.05, 14.11, 6.81 (d, J = 52.6 Hz).
³¹P-NMR (162 MHz, CDCl₃) δ = 35.05 (d, ²J_{P-F} = 64.6 Hz).
¹⁹F NMR (376 MHz, chloroform-*d*) δ ppm -212.30 (dd, *J*=65.2, 43.9 Hz, 1 F).

### Compound (IIb) :

74 g (400 mmol) of ethyl bromofluoroacetate are added slowly to 58.8 ml (400 mmol) of triethylphosphine dissolved in 400 ml of toluene. The reaction mixture is stirred overnight at room temperature then a few hours at 0°C before the compound (IIb) is filtered, washed with 80 ml of toluene and dried. Yield: 102 g (84 %).
Appearance : white solid (mp: 135-210°C).
¹H NMR (400MHz ,CDCl₃) δ = 7.47 (dd, *J* = 6.8, 43.3 Hz, 1 H), 4.25 (q, *J* = 7.1 Hz, 2 H), 2.65 (qddd, *J* = 7.7, 13.6, 15.6, 51.8 Hz, 6 H), 1.2.1 (t, *J* = 7.0 Hz, 3 H), 1.22 (td, *J*= 7.8, 19.1 Hz, 9 H).
¹³C-NMR (101 MHz, CDCl₃) δ =163.3 (dd, J = 1.5, 20.5 Hz), 83.60 (dd, J = 52.8, 206.9 Hz), 63.53, 13.55, 11.3 (d, J = 44.8 Hz), 5.6 (d, J = 5.9 Hz).
¹⁹F-NMR (377 MHz, CDCl₃) δ = -209.53 (d, ²J-F = 55.6 Hz).
³¹P-NMR (162 MHz, CDCl₃) δ = 44.07 (d, ²J_{P-F}= 56.7 Hz).

### Compound (IIc) (compound described in the literature: Thenappan, A.; Burton, D.J. J. Org. Chem. 1990, 55, 2311-2317) :

3.71 g (20 mmol) of ethyl bromofluoroacetate were added to a solution of 5 ml (20 mmol) of tributylphosphine in 50 ml of ethyl acetate. The solution was stirred overnight at room temperature before concentration under vacuum to afford the crude compound (IIc) as a semi-solid compound (crude yield: quantitative).
¹H NMR (400 MHz, chloroform-*d*) δ ppm 7.55 (dd, *J*=43.3, 6.5 Hz, 1 H), 4.42 (q, *J*=7.1 Hz, 2 H), 2.53 - 2.89 (m, 6 H), 1.62 - 1.74 (m, 6 H), 1.49 - 1.59 (m, 6 H), 1.39 (t, *J*=7.2 Hz, 3 H), 0.98 (t, *J*=7.3 Hz, 9 H)
¹³C NMR (101 MHz, chloroform-*d*) δ ppm 163.77 (dd, *J*=20.9, 1.8 Hz), 83.17 (dd, *J*=208.3, 53.5 Hz), 63.74, 23.74 (d, *J*=16.1 Hz), 23.43 (d, *J*=5.1 Hz), 17.94 (d, *J*=43.3 Hz), 13.85, 13.11
³¹P NMR (162 MHz, chloroform-*d*) δ ppm 38.94 (d, ²J_{P-F}=55.3 Hz).
¹⁹F NMR (376 MHz, chloroform-*d*) δ ppm -208.67 (dd, *J*=56.1, 43.4 Hz, 1 F).

### Compound (IId) :

4.57 g (24.7 mmol) of ethyl bromofluoroacetate were added to a solution of 5 g (24.7 mmol) oftriisobutylphosphine in 25 ml of THF. The solution was stirred 4 days at room temperature before concentration under vacuum. The residue was dried under high vacuum to form a solid. The solid was resuspended in a few ml of toluene, filtered, washed and dried under vacuum to afford 1.07 g of compound (IId) (yield: 11%).
Appearance : white solid (mp: 101°C).
¹H NMR (400MHz ,chloroform-d) δ = 7.64 (dd, *J*= 6.5, 42.6 Hz, 1 H), 4.39 - 4.07 (m, 2 H), 2.71 (ddd, *J =* 6.0, 13.4, 15.5 Hz, 3 H), 2.50 (ddd, *J*= 6.5, 14.2, 15.5 Hz, 3 H), 2.24 - 2.04 (m, 3 H), 1.24 (t, *J* = 7.2 Hz, 3 H), 1.03 (d, *J* = 6.8 Hz, 9 H), 1.00 (d, *J* = 6.5 Hz, 9 H)
¹³C NMR (101MHz ,chloroform-d) δ = 163.8 (dd, J = 2.5, 20.5 Hz), 83.2 (dd, J = 53.6, 210.5 Hz), 63.4, 27.8 (d, J = 38.9 Hz), 24.5, 24.4, 24.3, 24.2, 23.2 (d, J = 4.4 Hz), 13.6 ¹⁹F-NMR (377 MHz, CDCl₃) δ = -123.75 (d, ²J_{P-F} = 56.4 Hz).
³¹P-NMR (162 MHz, CDCl₃) δ = 37.82 (d, ²J_{P-F} = 56.7 Hz).
High-resolution MS (phosphonium cation) : calculated for C₁₆H₃₃FO₂P (phosphonium cation) : 307.2202; found : 307.2225.

### Compound (IIe) (compound described in the literature: Thenappan, A.; Burton, D.J. J. Org. Chem. 1990, 55, 2311-2317)

3.52 g (19 mmol) of ethyl bromofluoroacetate were added to a solution of 5 g (19 mmol) of triphenylphosphine in 15 ml of dichloromethane. The reaction mixture is stirred at room temperature before concentration under vacuum. The residue is resuspended in hot ethyl acetate - isopropanol (80 / 20) then filtered after cooling to room temperature and dried under vacuum to afford 4.54 g of compound (IIe) (yield: 54%).
Appearance : white solid.
¹H NMR (360 MHz, chloroform-*d*) δ ppm 9.71 (dd, *J*=41.7, 5.9 Hz, 1 H), 8.01 (dd, *J*=13.2, 8.1 Hz, 6 H), 7.79 - 7.89 (m, 3 H), 7.71 (td, *J*=7.9, 3.7 Hz, 6 H), 4.11 (q, *J*=7.2 Hz, 2 H), 1.00 (t, *J*=7.1 Hz, 3 H)
¹³C NMR (91 MHz, chloroform-*d*) δ ppm 163.34 (dd, *J*=21.5, 2.8 Hz, 1 C), 135.70 (d, *J*=3.5 Hz, 3 C), 134.75 (d, *J*=10.4 Hz, 6 C), 130.34 (d, *J*=13.1 Hz, 6 C), 114.53 - 115.85 (m, 3 C), 83.61 - 86.96 (m, 1 C), 63.60 (s, 1 C), 13.56 (s, 1 C)

### Compound (IIf) :

4.27 g (25 mmol) of methyl bromofluoroacetate were added to 25 ml (25 mmol) of a 1M solution of trimethylphosphine in toluene. The reaction mixture was stirred overnight at room temperature, filtered and the collected solid was washed with a few ml of toluene then dried under vacuum to afford 5.88 g of compound (IIf) as a white solid (yield: 95%).
Appearance : white solid (mp: 110°C).
¹H NMR (400MHz ,DMSO-d₆) δ = 6.88 (dd, *J*= 7.4, 43.4 Hz, 1 H), 3.87 (s, 3 H), 2.18 (d, *J*= 15.6 Hz, 9 H)
¹³C NMR (101 MHz, DMSO-*d*₆) δ ppm 163.51 (d, *J*=22.7 Hz), 83.82 (dd, *J*=199.5, 58.7 Hz), 54.01, 5.47 (d, *J*=51.4 Hz)
¹⁹F NMR (376MHz ,DMSO-d₆) δ = -212.44 (dd, J = 64.9, 43.6 Hz, 1 F)
³¹P NMR (162MHz ,DMSO-d₆) δ 35.75 (d, J = 65.2 Hz, 1 P)
High-resolution MS (phosphonium cation) : calculated for C₆H₁₃FO₂P (phosphonium cation) : 167.0637; found : 167.0645.

### Compound (IIg) :

10.65 g of t-butyl bromofluoroacetate were added to 50 ml (50 mmol) of a 1M solution of trimethylphosphine in toluene and the reaction mixture was stirred overnight at room temperature then a few hours at 0°C. The formed solid was filtered, washed with a few ml of toluene and dried under vacuum. 10.55 g of compound (IIg) were obtained (yield : 73 %).
Appearance : white solid (mp: 97.8°C).
¹H NMR (400MHz ,DMSO-d₆) δ = 6.69 (dd, *J* = 7.3, 43.6 Hz, 1 H), 2.14 (d, *J* = 15.4 Hz, 9 H), 1.51 (s, 9 H)
¹³C NMR (101MHz, ,DMSO-d₆) δ = 161.9 (d, J = 21.3 Hz), 86.4, 83.4 (dd, J = 200.3, 58.7 Hz), 27.5, 5.7 (d, J = 51.4 Hz)
¹⁹F NMR (376MHz ,DMSO-d₆) δ = -209.98 (dd, J = 65.6, 43.7 Hz, 1 F)
³¹P NMR (162MHz ,DMSO-d₆) δ = 35.17 (d, *J*= 65.2 Hz, 1 P)
High-resolution MS (phosphonium cation) : calculated for C₉H₁₉FO₂P (phosphonium cation) : 209.1107; found : 209.1111.

### Compound (IIh) :

100 ml (100 mmol) of a 1M solution of trimethylphosphine in toluene were added to a solution of 15.60 g (100 mmol) of bromofluoroacetamide in 150 ml of MeTHF. The reaction mixture was stirred overnight at room temperature then the compound (IIh) was filtered, washed with a few ml of toluene and dried under vacuum. Yield: 7.60 g (33 %).
Appearance : white solid (mp: 166.5°C).
¹H NMR (400MHz ,DMSO-d₆) δ = 8.33 (br. s., 1 H), 8.28 (br. s., 1 H), 6.49 (dd, *J*= 6.0, 44.8 Hz, 1 H), 2.08 (dd, *J* = 0.5, 15.4 Hz, 9 H)
¹³C NMR (101MHz ,DMSO-d₆) δ = 165.1 (d, J = 52.1 Hz), 84.9 (dd, J = 63.8, 209.1 Hz), 5.7 (d, J = 18.3 Hz)
¹⁹F NMR (376MHz ,DMSO-d₆) δ = -205.4 (dd, J = 44.9, 64.8 Hz, 1 F)
³¹P NMR (162MHz ,DMSO-d₆) δ = 65.2 (d, J = 65.2 Hz, 1 P)
High-resolution MS (phosphonium cation): measured: 152.069, theor.: 152.0641 Elemental analysis: calculated: C (25.88%), H (5.21%), N (6.04%) found: C (25.78%), H (5.24%), N (5.90%).

### Example 3

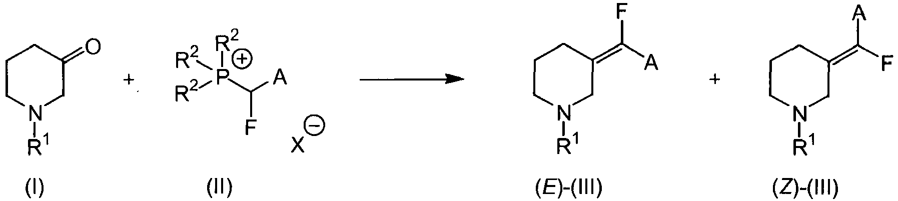

The results of the formation of the compounds (III) are reported in the table below.

| Compound (III) | | | From compounds | | Solvent | T° (°C) | % yield | E/Z ratio |
|---|---|---|---|---|---|---|---|---|
| | R¹ | A | (I) | (II) | | | | |
| (IIIa) | Bn | CO₂Et | (Ia) | (IIa) | Toluene | 0 | 96 | 80/20 |
| | | | | | Toluene | 60 | 71 | 70/30 |
| | | | | | Toluene | 40 | 77 | 75/25 |
| | | | | | Toluene | 22 | 87 | 76/24 |
| | | | | | Toluene | -20 | 56 | 83/17 |
| | | | | | Toluene | -33 | 49 | 84/16 |
| | | | | | DCM | 0 | 86 | 60/40 |
| | | | | | DMA | 0 | 55 | 67/33 |
| | | | | | MeCN | 0 | 80 | 56/44 |
| | | | | | AcOEt | 0 | 60 | 76/24 |
| | | | | | Heptanes | 0 | 32 | 74/26 |
| | | | | | CPME | 0 | 46 | 77/23 |
| | | | | | MeCy | 0 | 36 | 74/26 |
| | | | | | THF | 0 | 34 | 78/22 |
| | | | | | DIPE | 0 | 40 | 74/26 |
| | | | | | Anisole | 0 | 68 | 72/28 |
| | | | | | PhF | 0 | 44 | 72/28 |
| | | | | | DBE | 0 | 52 | 76/24 |
| | | | | | PhCl | 0 | 45 | 73/27 |
| | | | (Ia) | (IIb) | Toluene | 0 | 72 | 76/24 |
| | | | (Ia) | (IIc) | Toluene | 0 | 52 | 73/27 |
| | | | (Ia) | (IId) | Toluene | 0 | 49 | 71/29 |
| | | | (Ia) | (IIe) | Toluene | 0 | 3 | 67/33 |
| (IIIb) | Me | CO₂Et | (Ib) | (IIa) | Toluene | 0 | 35 | 96/4 |
| (IIIc) | Et | CO₂Et | (Ic) | (IIa) | Toluene | 0 | 65 | 84/16 |
| (IIId) | Allyl | CO₂Et | (Id) | (IIa) | Toluene | 0 | 40 | 90/10 |
| (IIIe) | 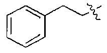 | CO₂Et | (Ie) | (IIa) | Toluene | 0 | 86 | 93/7 |
| (IIIf) | 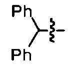 | CO₂Et | (If) | (IIa) | Toluene | 0 | 85 | 67 / 33 |
| (IIIg) | H | CO₂Et | (Ig) | (IIa) | Toluene | 0 | | |
| (IIIh) | 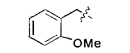 | CO₂Et | (Ih) | (IIa) | Toluene | 0 | 58 | 81 / 19 |
| (IIIi) | 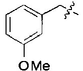 | CO₂Et | (Ii) | (IIa) | | | | |
| (IIIj) | 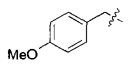 | CO₂Et | (Ij) | (IIa) | | | | |
| (IIIk) | Bn | CO₂Me | (Ia) | (IIf) | Toluene | 0 | 76 | 83 / 17 |
| (III1) | Bn | CO₂tBu | (Ia) | (IIg) | Toluene | 0 | 32 | 58/42 |
| (IIIm) | Bn | CONH₂ | (Ia) | (IIh) | Toluene | 0 | | |
| (IIIn) | Bn | CO₂H | (Ia) | (IIi) | | | | |
| (IIIo) | Bn | CN | (Ia) | (IIj) | | | | |

### Compound (IIIa) :

30 g (124 mmol) of 94 w/w% compound (Ia) . HCl salt are suspended in 124 ml of toluene. 124 ml of water and 13.14 g of sodium carbonate are added and the resulting mixture is stirred a few minutes at room temperature before decantation. The two layers are separated and the organic one is dried over sodium sulfate then filtered to give 142 g of solution of 16.6 w/w% of compound (Ia) in toluene. The so-obtained solution of compound (Ia) is added to a suspension of 38.8 g (148 mmol) of compound (IIa) at 0°C. 22.5 g (148 mmol) of DBU are added and the reaction mixture is stirred overnight at 0°C before being quenched with 62 ml of water. The two layers are separated and the organic one is washed with 62 ml of water, dried over sodium sulfate and filtered to give 251.7 g of 12.9 w/w% of compound (IIIa) solution in toluene that is used as such in the next step. (yield: 96%, E / Z ratio: 80 / 20).

The same procedure was used in different solvents and at different temperatures.

A solution of 50.5 mmol of compound (IIIa) in toluene is concentrated under vacuum and the residue is purified by chromatography to give 11.36 g of compound (IIIa) (mixture of (*E*)- and (*Z*)-isomer) as an oil with a limited stability. Yield: 81 %.

5.56 g of p-toluenesulfonic acid monohydrate are added to a solution of 8.12 g of compound (IIIa) in MIBK and the reaction mixture is warmed up to complete dissolution then cooled to room temperature. 12 g of tosylate salt of compound (IIIa) is obtained as a stable solid after filtration, washing and drying under vacuum (E / Z ratio: 85 / 15).

12 g of compound (IIIa) are purified by preparative HPLC on ChiralPak AD column to deliver 4.16 g of compound (*E*)-(IIIa) and 820 mg of compound (2)-(IIIa).

2.85 g (15.0 mmol) of p-toluenesulfonic acid monohydrate are added to 4.16 g (15 mmol) of compound (*E*)-(IIIa) dissolved in 34 ml of MIBK. The mixture is warmed up until the salt is dissolved, filtered hot then let cooled down to room temperature. The so-obtained solid is filtered, washed with a few ml of MIBK and dried. 5.23 g of compound (*E*)-(IIIa) .TsOH salt are obtained as a white solid (Yield: 78 %).

0.57 g (3 mmol) of p-toluenesulfonic acid monohydrate are added to 820 mg (2.96 mmol) of compound (*Z*)-(IIIa) dissolved in 10 ml of MIBK and 3 ml of ethanol. The mixture is warmed up until the salt is dissolved, filtered hot then let cooled down to room temperature. The so-obtained solid is filtered, washed with a few ml of MIBK and dried. 790 mg of compound (*Z*)-(IIIa) .TsOH salt are obtained as a white solid (Yield: 59 %).

### Compound (E)-(IIIa) :

Appearance : pale yellow liquid, darkens rapidly.
¹H NMR (361 MHz, chloroform-*d*) δ ppm 7.17 - 7.46 (m, 5 H), 4.19 (q, J = 7.0 Hz, 2 H), 3.56 - 3 .66 (m, 4 H), 2.52 - 2.55 (m, 2 H), 2.37 - 2.40 (m, 2 H), 1.69 - 1.75 (m, 2 H), 1.22 (t, J = 7.1 Hz, 3 H)
¹⁹F NMR (377 MHz, chloroform-d) δ = -128.28 (s, 1 F).

### Compound (E)-(IIIa) . TsOH salt :

Appearance : white solid (mp: 103.5°C).
¹H NMR (400MHz , chloroform-d) δ = 10.66 (br. s., 1 H), 7.74 (d, *J* = 8.1 Hz, 2 H), 7.52 (d, *J*= 7.1 Hz, 2 H), 7.44 - 7.36 (m, 1 H), 7.36 - 7.30 (m, 2 H), 7.16 (d, *J*= 8.1 Hz, 2 H), 4.85 (d, *J* = 13.6 Hz, 1 H), 4.3:1 (d, *J*= 5.3 Hz, 2 H), 4.20 - 4.04 (m, 2 H), 3.89 (ddd, *J*= 4.2, 8.1,13.7 Hz, 1 H), 3.58 - 3.44 (m, 1 H), 3.22 - 3.04 (m, 1 H), 2.72 (td, *J* = 4.2, 14.5 Hz, 1 H), 2.35 (s, 3 H), 2.25 (tdd, *J* = 4.3, 10.3, 14.5 Hz, 1 H), 2.14 - 1.98 (m, 1 H), 1.95 - 1.82 (m, 1 H), 1.18 (t, *J* = 7.2 Hz, 3 H)
¹³C NMR (101 MHz, chloroform-*d*) δ ppm 159.68 (d, *J*=35.2 Hz), 145.06 (d, *J*=259.7 Hz), 142.57, 139.91, 131.17, 129.90, 129.15, 128.72, 128.41, 125.79, 121.84 (d, *J*=19.8 Hz), 61.96, 59.27, 50.85, 49.40 (d, *J*=5.1 Hz), 22.74 (d, *J*=8.1 Hz), 21.21, 20.85, 13.79. ¹⁹F NMR (376 MHz, chloroform-*d*) δ ppm -118.96 (br. s., 1 F)
LC-high resolution MS: calculated for C₁₆H₂₁FNO₂ (compound E-(IIIa) + H): 287.1551; found: 278.1563
Elemental analysis: calculated for C₂₃H₂₈FNO₅S: C (61.45%), H (6.28%), N (3.12%); found: C (60.67%), H (6.24%), N (3.14%)

### Compound (Z)-(IIIa) :

Appearance : pale yellow liquid, darkens rapidly.
¹H NMR (400 MHz, chloroform-*d*) δ ppm 7.22 - 7.34 (m, 5 H), 4.28 (q, J = 7.2 Hz, 2H), 3.62 (s, 2 H), 3.25 (d, J = 3.0 Hz, 2 H), 2.73 - 2.80 (m, 2 H), 2.51 - 2.58 (m, 2 H), 1.72 (dt, J = 11.5, 5.5 Hz, 2 H), 1.33 (t, J = 7.0 Hz, 3 H)
¹³C NMR (101 MHz, chloroform-*d*) δ ppm 161.40 (d, *J*=35.2 Hz,), 141.78 (d, *J*=250.9 Hz), 137.38 (d, *J*=2.9 Hz), 129.14, 128.30, 127.26, 62.45, 61.18, 52.84, 51.88 (d, *J*=11.7 Hz), 25.46 (d, *J*=1.5 Hz), 24.95,14.14
¹⁹F NMR (377MHz ,chloroform-d,) δ = -128.47 (s, 1 F)

### Compound (Z)-(IIIa) . TsOH :

Appearance : white solid (mp: 167.1°C)
¹H NMR (400 MHz, chloroform-*d*) δ ppm 10.63 (br. s., 1 H), 7.74 (d, *J*=8.3 Hz, 2 H), 7.52 (d, *J*=6.8 Hz, 2 H), 7.34 - 7.42 (m, 1 H), 7.26 - 7.34 (m, 2 H), 7.15 (d, *J*=7.8 Hz, 2 H), 4.33 (d, *J*=5.3 Hz, 2 H), 4.20 - 4.28 (m, 3 H), 4.17 (d, *J*=13.6 Hz, 1 H), 3.70 (ddd, *J*=13.6, 7.7, 2.6 Hz, 1 H), 3.39 - 3.53 (m, 1 H), 3.08 - 3.30 (m, 2 H), 2.43 - 2.59 (m, 1 H), 2.35 (s, 3 H), 1.94 - 2.09 (m, 1 H), 1.87 (s, 1 H), 1.30 (t, *J*=7.2 Hz, 3 H).
¹³C NMR (101 MHz, chloroform-*d*) δ ppm 159.83 (d, *J*=35.2 Hz), 144.46 (d, *J*=263.4 Hz), 142.50, 139.92, 131.16, 129.91, 129.15, 128.72, 128.32, 125.79, 121.54 (d, *J*=10.3 Hz), 61.80, 59.19, 50.84, 48.35 (d, *J*=11.7 Hz), 23.07, 21.20, 21.16, 13.89.
¹⁹F NMR (376 MHz, chloroform-*d*) δ ppm -120.65 (br. s., 1 F)
LC-high resolution MS: calculated for C₁₆H₂₁FNO₂ (compound (*Z*)-(IIIa) + H): 287.1551; found: 278.1559
Elemental analysis: calculated for C₂₃H₂₈FNO₅S: C (61.45%), H (6.28%), N (3.12%); found: C (60.91%), H (6.34%), N (3.15%)

### Compound (IIIb) :

1.0 g (6.68 mmol) of compound (Ib).HCl salt, 2.09 g (8.02 mmol) of compound (IIa) and 924 mg (6.68 mmol) of potassium carbonate were suspended in 13 ml of toluene. After cooling to 0°C, 1.21 ml (8.02 mmol) of DBU were added and the reaction mixture was stirred overnight at 0°C. 13 ml of water were added to the reaction mixture and the two layers were separated after a few minutes of stirring. The water layer was extracted with 13 ml of toluene and the combined organic ones were dried over sodium sulfate and filtered to afford 50.6 g of a 0.94 w/w% solution of compound (IIIb) in toluene (in situ yield: 35 %). The solution was concentrated under vacuum and the residue was purified by filtration through a pad of silica gel (eluent: acetone) to afford 404 mg of purified product (yield: 30 %). E / Z ratio: 96/4.
Appearance : pale orange oil.

### Compound (E)-(IIIb) :

¹H NMR (400 MHz, chloroform-*d*) δ ppm 4.27 (q, *J*=7.2 Hz, 2 H), 3.52 (d, *J*=2.0 Hz, 2 H), 2.47 - 2.51 (m, 2 H), 2.34 - 2.39 (m, 1 H), 2.34 (s, 3 H), 1.71 - 1.79 (m, 2 H), 1.34 (t, *J*=7.1 Hz, 3 H)
¹³C NMR (101 MHz, chloroform-*d*) δ ppm 161.04 (d, *J*=35.9 Hz, 1 C), 142.15 (d, *J*=250.9Hz, 1 C), 130.39 (d, *J*=13.9 Hz, 1 C), 61.21, 55.40, 54.36 (d, *J*=5.1 Hz, 1 C), 46.13 (s, 1 C), 24.90 (d, *J*=2.2 Hz, 1 C), 24.53 (d, *J*=8.8 Hz, 1 C), 14.08
¹⁹F NMR (376 MHz, chloroform-*d*) δ ppm -129.72 (br. s., 1 F)
High resolution MS: calculated for C₁₀H₁₇FNO₂ (Compound (IIIb) + H): 202.1238; found: 202.1225

### Compound (Z)-(IIIb) :

¹H NMR (400 MHz, chloroform-*d*) δ ppm 3.12 (d, *J*=3.0 Hz, 2 H)
¹³C NMR (101 MHz, chloroform-*d*) δ ppm 61.13, 55.49, 53.78 (d, *J*=11.7 Hz, 1 C), 25.29 (d, *J*=2.2 Hz, 1 C)
¹⁹F NMR (376 MHz, chloroform-*d*) δ ppm -129.12 (br. s., 1 F)

### Compound (IIIc) :

The compound (IIIc) was obtained from the compounds (Ic) .HCl salt and (IIa) using the same procedure as for the compound (IIIa). Yield: 65%. E / Z ratio: 85 / 15.
Appearance : pale yellow liquid, darkens rapidly.

### Compound (E)-(IIIc) :

¹H NMR (360 MHz, chloroform-*d*) δ ppm 1.13 (t, *J*=7.32 Hz, 3 H) 1.34 (t, *J*=7.14 Hz, 3 H) 1.77 (dd, *J*=6.59, 5.12 Hz, 2 H) 2.40 (td, *J*=6.40, 2.56 Hz, 2 H) 2.48 - 2.56 (m, 2 H) 2.60 (d, *J*=5.49 Hz, 2 H) 3.62 (d, *J*=1.46 Hz, 2 H) 4.28 (q, *J*=7.32 Hz, 3 H)

### Compound (Z)-(IIIc) :

¹H NMR (360 MHz, chloroform-*d*) δ ppm 1.13 (t, *J*=7.32 Hz, 3 H) 1.34 (t, *J*=7.14 Hz, 3 H) 1.77 (dd, *J*=6.59, 5.12 Hz, 2 H) 2.52 (q, *J*=7.32 Hz, 2 H) 2.56 - 2.62 (m, 2 H) 2.73 - 2.81 (m, 2 H) 3.21 (d, *J*=2.93 Hz, 2 H) 4.28 (q, *J*=7.32 Hz, 2 H)

### Compound (IIId) :

The compound (IIId) was obtained from the compound (Id) .HCl salt and (IIa) using the same procedure than for the compound (IIIa). Yield: 40%, E / Z ratio: 90/10 Appearance : pale yellow liquid, darkens rapidly.

### Compound (E)-(IIId) :

¹H NMR (400 MHz, chloroform-*d*) δ ppm 1.33 (t, *J*=7.05 Hz, 3 H) 1.68 - 1.81 (m, 2 H) 2.33 - 2.46 (m, 2 H) 2.50 - 2.61 (m, 2 H) 3.02 - 3.14 (m, 2 H) 3.57 (d, *J*=2.01 Hz, 2 H) 4.27 (d, *J*=7.05 Hz, 2 H) 5.12 - 5.29 (m, 2 H) 5.77 - 5.99 (m, 1 H)
¹³C NMR (91 MHz, chloroform-*d*) δ ppm 14.42 (s, 1 C) 20.91 (s, 1 C) 23.50 (d, *J*=7.61 Hz, 1 C) 49.69 (d, *J*=4.84 Hz, 1 C) 50.99 (s, 1 C) 58.28 (s, 1 C) 62.67 (s, 1 C) 125.91 (s, 1 C) 127.17 (s, 1 C) 145.65 (d, *J*=260.90 Hz, 1 C) 160.47 (d, *J*=31.80 Hz, 1 C)
¹⁹F NMR (377MHz, chloroform-d,) δ = -128.85 (s, 1 F)

### Compound (IIIe) :

The compound (IIIe) was obtained from the compound (Ie) .HCl salt and (IIa) using the same procedure than for the compound (IIIa). Yield: 86%. E / Z ratio: 93 / 7.
Appearance : pale yellow liquid, darkens rapidly.

### Compound (E)-(IIIe):

¹H NMR (400MHz, chloroform-d) δ = 7.28 - 7.24 (m, 2 H), 7.19 - 7.15 (m, 3 H), 4.26 (q, *J* = 7.2 Hz, 2 H), 3.69 (d, *J*= 2.0 Hz, 2 H), 2.85 - 2.81 (m, 2 H), 2.68 - 2.63 (m, 2 H), 2.63 - 2.60 (m, 2 H), 2.38 (dt, *J*= 2.6, 6.5 Hz, 2 H), 1.77 - 1.73 (m, 2 H), 1.31 (t, *J* = 7.1 Hz, 3 H).
¹³C NMR (101MHz, chloroform-d) δ = 160.9 (d, J = 35.4 Hz), 142.0 (d, J = 251.3 Hz), 139.9, 130.3 (d, J = 13.4 Hz), 128.5, 128.1, 125.8, 61.0, 59.8, 53.3, 52.1 (d, J = 4.6 Hz), 33.6, 25.0 (d, J = 8.1 Hz), 24.6 (d, J = 1.7 Hz), 13.9.
¹⁹F NMR (377MHz , chloroform-d,) δ = -128.85 (s, 1 F).

### Compound (Z)-(IIIe):

¹H NMR (400MHz, chloroform-d, signals for Z-isomer) δ = 7.28 - 7.24 (m, 2 H), 7.19 - 7.15 (m, 3 H), 4.25 (q, *J*= 7.1 Hz, 2 H), 3.26 (d, *J* = 3.0 Hz, 2 H), 2.85 - 2.81 (m, 2 H), 2.80 - 2.73 (m, 4 H), 2.68 - 2.63 (m, 2 H), 1.77 - 1.71 (m, 2 H), 1.31 (t, *J* = 7.1 Hz, 3 H).
¹³C NMR (101MHz, chloroform-d, signals for Z-isomer) δ = 161.1 (d, J = 35.8 Hz), 141.5 (d, J = 250.9 Hz), 139.9, 130.1 (d, J = 11.7 Hz), 128.5, 128.1, 125.8, 60.9, 59.8, 53.2, 51.7 (d, J = 11.6 Hz), 33.5, 25.4 (d, J = 1.5 Hz), 24.6 (d, J = 1.7 Hz), 13.9.
¹⁹F NMR (377MHz, chloroform-d, signals for Z-isomer) δ = -128.48 (s, 1 F).

### Compound (IIIf) :

The compound (IIIf) was obtained from the compound (If) .HCl salt and (IIa) using the same procedure than the one used for the compound (IIIa).
Appearance : pale yellow liquid, darkens rapidly.

### Compound (IIIg) :

The compound (IIIg) was obtained from the compounds (Ig) .HCl salt and (IIa) using the same procedure than the one used for the compound (IIIa).
Appearance : pale yellow liquid, darkens rapidly.

### Compound (IIIh) :

The compound (IIIh) was obtained from the compound (Ih) .HCl salt and (IIa) using the same procedure than the one used for the compound (IIIa).
Appearance : pale yellow liquid, darkens rapidly.

### Compound (E)-(IIIh) :

¹H NMR (360 MHz, chloroform-*d*) δ ppm 7.32 (dd, *J*=7.5, 1.6 Hz, 2 H), 7.23 (td, *J*=7.9, 1.8 Hz, 2 H), 6.92 (td, *J*=7.5, 1.1 Hz, 2 H), 6.86 (d, *J*=8.4 Hz, 2 H), 4.20 (q, *J*=7.3 Hz, 2 H), 3.81 (s, 3 H), 3.62 - 3.68 (m, 4 H), 2.54 - 2.63 (m, 2 H), 2.37 (td, *J*=6.5, 2.7 Hz, 2 H), 1.68 - 1.79 (m, 2 H), 1.24 (t, *J*=7.1 Hz, 3 H)
¹³C NMR (91 MHz, chloroform-*d*) δ ppm 160.97 (d, *J*=36.7 Hz, 1 C), 157.73 (s, 1 C), 142.06 (d, *J*=247.7 Hz, 1 C), 130.62 (s, 1 C), 128.07 (s, 1 C), 125.67 (s, 1 C), 120.16 (s, 1 C), 110.29 (s, 1 C), 61.04 (s, 1 C), 55.51 (s, 1 C), 55.28 (s, 1 C), 52.76 (s, 1 C), 52.47 (d, *J*=4.8 Hz, 1 C), 25.00 (d, *J*=8.3 Hz, 1 C), 24.59 (d, *J*=2.1 Hz, 1 C), 13.95 (s, 1 C)

### Compound (IIIK) :

The compound (IIIk) was obtained from the compound (Ia) .HCl salt and (IIf) using the same procedure than for the compound (IIIa).
Appearance : pale yellow liquid, darkens rapidly.

### Compound (E)-(IIIk) :

¹H NMR (600 MHz, chloroform-*d*) δ ppm 7.28 - 7.34 (m, 4 H), 7.23 - 7.28 (m, 1 H), 3.70 (s, 3 H), 3.59 (br. s, 4 H), 2.48 - 2.56 (m, 2 H), 2.39 (td, *J*=6.4, 2.6 Hz, 2 H), 1.67 - 1.76 (m, 2 H)
¹³C NMR (151 MHz, chloroform-*d*) δ ppm 161.50 (d, *J*=36.2 Hz), 142.05 (d, *J*=248.1 Hz), 137.69, 130.82 (d, *J*=13.2 Hz), 129.25, 128.21, 127.16, 62.61, 52.78, 52.63 (d, *J*=5.5 Hz), 51.93, 25.14 (d, *J*=8.8 Hz), 24.65.
High resolution MS: calculated for C₁₅H₁₈FNO₂ (M+°): 263.1322, found: 263.1325.

### Compound (Z)-(IIIk):

¹H NMR (600 MHz, chloroform-*d*) δ ppm 7.28 - 7.34 (m, 5 H), 3.81 (s, 3 H), 3.59 (s, 2 H), 3.22 (d, *J*=3.0 Hz, 2 H), 2.76 (td, *J*=6.4, 1.9 Hz, 2 H), 2.48 - 2.56 (m, 2 H), 1.67 - 1.76 (m, 2 H).
¹³C NMR (151 MHz, chloroform-*d*) δ ppm 161.85 (d, *J*=35.1 Hz), 141.50 (d, *J*=250.3 Hz), 137.64, 130.97 (d, *J*=12.1 Hz), 129.06, 128.27, 127.18, 62.58, 52.89, 52.63 (d, *J*=5.5 Hz), 52.00, 25.52 (d, *J*=2.2 Hz), 25.09.
High resolution MS: calculated for C₁₅H₁₈FNO₂ (M+°): 263.1322, found: 263.1328.

### Experiment 4

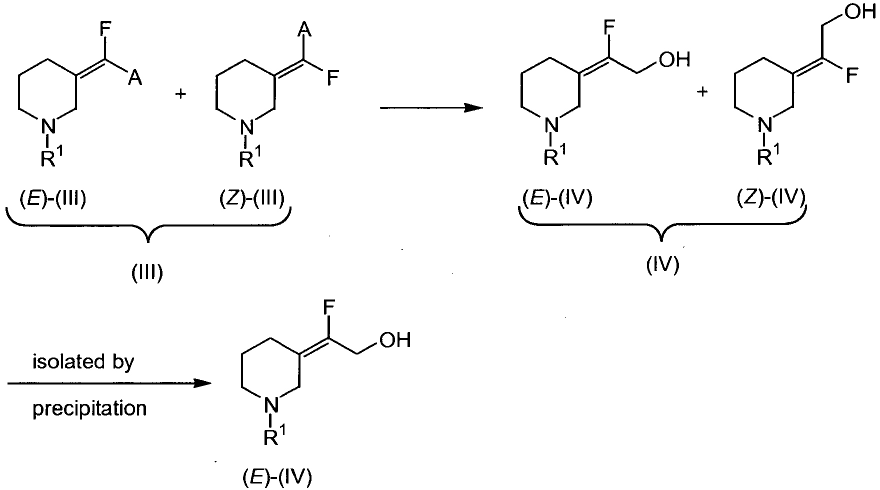

| Compound | R¹ |
|---|---|
| (IVa) | Bn |
| (IVb) | Me |
| (IVc) | Et |
| (IVd) | Allyl |
| (IVe) | 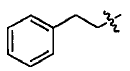 |
| (IVf) | 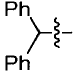 |
| (IVg) | H |
| (IVh) | 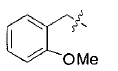 |
| (IVi) | 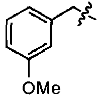 |
| (IVj) | 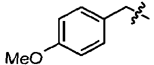 |

### Compound (E)-(IVa):

437 mmol of compound (IIIa) in solution in toluene is cooled to 0°C. 212 g (682 mmol) of 65 w/w% sodium bis(2-methoxyethoxy)aluminumhydride (Red-Al) solution in toluene are added and the reaction mixture is stirred 1 hour at 0°C the excess of Red-Al is quenched with 77 ml of acetone and the reaction mixture is allowed to warm to room temperature. 568 ml of water and 235 ml of 50 w/w% sodium hydroxide aqueous solution are added and the resulting mixture is warmed to 50°C before decantation. The two layers are separated and the organic one is washed with 437 ml of water, dried over sodium sulfate, filtered and concentrated under vacuum. 1170 ml of heptanes are added to the oily residue and the resulting solid is filtered and recrystallized from 188 ml of diisopropyl ether to give 53.5 g of compound (E)-(IVa). Yield: 52%, E / Z ratio > 99 / 1.
Appearance : solid (mp: 92.0°C).
¹H NMR (360 MHz, chloroform-*d*) δ ppm 1.51 - 1.71 (m, 2 H) 2.20 - 2.30 (m, 2 H) 2.51 (t, *J*=5.90 Hz, 2 H) 2.96 (d, *J*=1.46 Hz, 2 H) 3.54 (s, 2 H) 4.15 (d, *J*=23.05 Hz, 2 H) 7.13-7.36 (m, 5H)
¹³C NMR (91 MHz, chloroform-*d*) δ ppm 24.18 (d, *J*=7.61 Hz, 1 C) 25.39 (s, 1 C) 53.97 (d, *J*=7.61 Hz, 1 C) 54.15 (s, 1 C) 57.87 (d, *J*=31.83 Hz, 1 C) 63.21 (s, 1 C) 116.05 (d, *J*=16.61 Hz, 1 C) 127.73 (s, 1 C) 128.75 (s, 2 C) 129.69 (s, 2 C) 137.85 (s, 1 C) 152.75 (dd, *J*=248.43, 1.00 Hz, 1 C)
¹⁹F NMR (377 MHz, chloroform-*d*) δ ppm -120.14 (s, 1 F)
Elemental analysis: calculated: C (71.46%), H (7.71%), F (8.07%), N (5.95%), O (%); found: C (71.38%), H (7.91%), N (5.99%)

### Compound (E)-(IVe):

5.6 ml (19.5 mmol) of a 70 w/w% solution of sodium bis(2-methoxyethoxy)aluminumhydride (Red-Al) in toluene were added to 38 g (10.2 mmol) of a 7.8 w/w% solution of compound (IIIe) in toluene kept at 0°C. After an hour at 0°C, the excess of Red-Al was quenched with 1.2 ml (16.4 mmol) of acetone and the reaction mixture was stirred overnight at room temperature. The reaction mixture was warmed to 50°C and 14 ml of water and 5.5 ml (105 mmol) of a 50 w/w% aqueous solution of sodium hydroxide were added. The layers were separated and the organic one was washed with 14 ml of water, dried over sodium sulfate and filtered. The filtrate was evaporated under vacuum to dryness to give 2.40 g of crude compound (IVe) (LC-assay: 94.1 w/w%, E / Z ratio: 92 / 8, yield: 89%). The crude product was recrystallized from 6 ml of DBE to afford 1.87 g of compound (*E*)-(IVe) as a solid product (yield: 73 %, E / Z ratio: 98 / 2). LC analysis of the mother-liquor revealed a E / Z ratio of 40 / 60, as a proof of the selective precipitation of the compound (*E*)-(IVe).
Appearance : tanned solid (mp: 110.5°C).
¹H NMR (400 MHz, chloroform-*d*) δ ppm 7.25 - 7.32 (m, 2 H), 7.15 - 7.23 (m, 3 H), 4.20 (d, *J*=23.2 Hz, 2 H), 3.89 (br. s., 1 H), 3.04 (d, *J*=1.3 Hz, 2 H), 2.76 - 2.86 (m, 2 H), 2.55 - 2.68 (m, 4 H), 2.27 (td, *J*=6.2, 1.8 Hz, 2 H), 1.62 - 1.75 (m, 2 H)
¹³C NMR (101 MHz, chloroform-*d*) δ ppm 152.80 (d, *J*=247.9 Hz), 139.94, 128.61, 128.42, 126.11, 115.20 (d, *J*=16.1 Hz), 60.27, 57.04 (d, *J*=31.5 Hz), 53.88 (d, *J*=8.1 Hz), 53.81, 33.39, 25.06 (d, *J*=1.5 Hz), 23.76 (d, *J*=8.1 Hz)
¹⁹F NMR (376 MHz, chloroform-*d*) δ ppm -119.40 (t, *J*=23.0 Hz, 1 F)
High resolution MS: calculated for C₁₅H₂₁NOF (M + H): 250.1607, found: 250.1604. Elemental analysis: calculated: C (72.26%), H (8.09%), F (7.62%), N (5.62%), O (6.42%); found: C (71.29%), H (8.20%), N (5.51%)

### Experiment 5

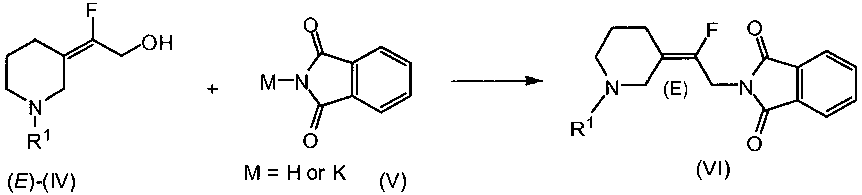

| Compound | R¹ |
|---|---|
| (VIa) | Bn |
| (VIb) | Me |
| (VIc) | Et |
| (VId) | Allyl |
| (VIe) | 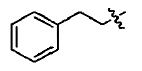 |
| (VIf) | 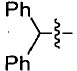 |
| (VIg) | H |
| (VIh) | 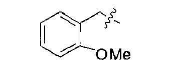 |
| (VIi) | 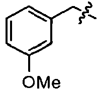 |
| (VIj) | 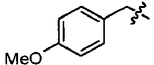 |

### Compound (VIa) via the mesylate of the Compound (IIIa):

28.2 g (120 mmol) of compound (*E*)-(IVa) and 18.4 ml (132 mmol) of triethylamine are dissolved in 180 ml of toluene at 0°C. 14.4 g (125.8 mmol) of methanesulfonyl chloride are added slowly and the reaction mixture is stirred 1 hour at 0°C. The formed triethylamine hydrochloride is washed out with 120 ml of cold water and the organic layer is dried over sodium sulphate, filtered and added to a suspension of 22.2 g (120 mmol) of potassium phtalimide (compound (V), M = K) and 2.43 g (6.6 mmol) of TBAI in 60 ml of toluene. The reaction mixture is stirred overnight at room temperature than washed with 120 ml of water, filtered and concentrated under vacuum. The residue is crystallized from 30 ml of isopropanol to give 33 g of a white solid. Yield: 76%.
Appearance : white solid (mp: 125.1°C).
¹H NMR (360 MHz, chloroform-*d*) δ ppm 1.47 - 1.76 (m, 4 H) 2.20 - 2.28 (m, 2 H) 2.47 - 2.56 (m, 2 H) 3.22 (d, *J*=1.46 Hz, 2 H) 3.63 (s, 2 H) 4.40 (d, *J*=20.90 Hz, 2 H) 7.12 - 7.42 (m, 5 H) 7.72 (dd, *J*=5.49, 3.29 Hz, 2 H) 7.85 (dd, *J*=5.49, 2.93 Hz, 2 H) ¹³C NMR (91 MHz, chloroform-*d*) δ ppm 24.14 (s, 1 C) 25.24 (d, *J*=1.38 Hz, 1 C) 35.39 (d, *J*=32.52 Hz, 1 C) 53.94 (s, 1 C) 54.35 (d, *J*=7.61 Hz, 1 C) 63.31 (s, 1 C) 117.42 (d, *J*=12.46 Hz, 1 C) 123.83 (s, 1 C) 125.73 (s, 1 C) 127.53 (s, 1 C) 128.68 (d, *J*=4.15 Hz, 2 C) 129.46 (s, 1 C) 129.67 (s, 1 C) 132.47 (s, 1 C) 134.45 (s, 254 C) 134.26 - 134.58 (m, 2 C) 138.46 (s, 1 C) 147.70 (d, *J*=248.43 Hz, 1 C) 168.04 (s, 1 C) ¹⁹F NMR (377 MHz, chloroform-*d*) δ ppm -118.91 (s, 1 F)
Elemental analysis: calculated: C (72.51 %), H (5.81 %), F (5.21 %), N (7.69%), O (8.78%); found: C (72.68%), H (5.84%), N (7.66%)

### Compound (VIa) via a Mitsunobu reaction :

8.43 ml (42.5 mmol) of DIAD were added dropwise to a cold (-10°C) solution of 10 g (42.5 mmol) of compound (*E*)-(IVa), 6.25 g (42.5 mmol) of phtalimide and 11.15 g (42.5 mmol) of triphenylphosphine in 85 ml of toluene. After 24 hours at -10°C, water was added. The insoluble materials were filtered off and the filtrate was allowed to decante. The two layers were separated and the organic one was dried over sodium sulfate, filtered and concentrated under vacuum. The residue was recrystallized from isopropanol to afford 10.2 g of compound (VIa) (yield: 66%).
NMR and MS spectra identical to those of the compound (VIa) obtained via the mesylate of the compound (IIIa).

### Compound (VIe) via the mesylate of the Compound (IIIe) :

0.49 ml (6.32 mmol) of methanesulfonyl chloride are added over 10 minutes to a solution of 1.50 g (6.02 mmol) of compound (IVe) and 0.92 ml (6.62 mmol) of triethylamine in 9 ml of toluene kept at 0°C. After 1 hour at 0°C, 6 ml of water were added. The layers were separated and the organic one was dried over sodium sulfate and filtered. The so-obtained solution of mesylate was added over 5 minutes to a suspension of 1.17 g (6.32 mmol) of potassium phtalimide (compound (V), M = K) and 133 mg (0.36 mmol) of TBAI in 3 ml of toluene. The reaction mixture was stirred at 10°C for 2 hours then overnight at room temperature. 6 ml of water were added, the insoluble materials were filtered off, the filtrate was allowed to decant, the water layer was discarded and the organic one was dried over sodium sulfate and filtered to afford 25.12 g of a 4.6 w/w% solution of compound (VIe) in toluene (in situ yield: 51%). The solution was concentrated under vacuum and the residue was recrystallized from n-butanol to afford 1.02 g of compound (VIe). The mother-liquor was concentrated under vacuum and filtered through a pad of silica gel (eluent: heptanes - ethyl acetate 1 / to 1 / 3) to yield 0.11 g more of compound (VIe). Combined yield : 47%.
Appearance : white solid (mp: 97.0°C).
¹H NMR (600 MHz, chloroform-*d*) δ ppm 7.85 (dd, *J*=5.3, 3.0 Hz, 2 H), 7.72 (dd, *J*=5.7, 3.0 Hz, 2 H), 7.28 - 7.32 (m, 2 H), 7.24 - 7.27 (m, 2 H), 7.21 (t, *J*=7.2 Hz, 1 H), 4.50 (d, *J*=21.9 Hz, 2 H), 3.29 (s, 2 H), 2.89 - 2.94 (m, 2 H), 2.69 - 2.74 (m, 2 H), 2.59 - 2.63 (m, 2 H), 2.26 (t, *J*=5.5 Hz, 2 H), 1.63 - 1.73 (m, 2 H)
¹³C NMR (151 MHz, chloroform-*d*) δ ppm 167.60, 147.56 (d, *J*=247.0 Hz), 140.27, 134.04, 131.98, 128.71, 128.39, 126.03, 123.41, 117.02 (d, *J*=15.4 Hz), 60.50, 53.95, 53.84 (d, *J*=7.7 Hz), 34.99 (d, *J*=32.9 Hz), 33.73, 24.92, 23.71 (d, *J*=7.7 Hz)
¹⁹F NMR (377MHz ,chloroform-d,) δ = -117.73 (s, 1 F).
High resolution MS: calculated for C₂₃H₂₄N₂O₂F (M + H): 379.1822, found: 379.1820. Elemental analysis: calculated: C (73.00%), H (6.13%), F (5.02%), N (7.40%), O (8.46%); found: C (72.53%), H (6.42%), N (7.32%)

### Experiment 6

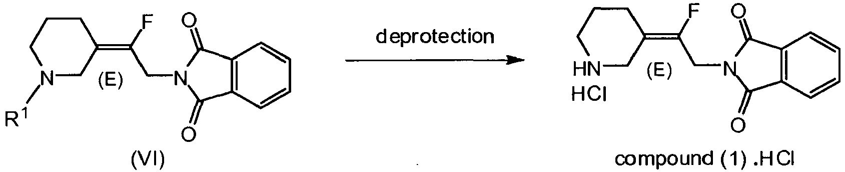

### Compound (1) .HCl salt from Compound (VIa) :

9.8 ml (90.6 mmol) of 1-chloroethyl chloroformate are added slowly to a solution of 30 g (82.3 mmol) of compound (*E*)-(Va) in 165 ml of toluene kept at 0°C. The reaction mixture is stirred 1 hour at room temperature than 1 hour at 80°C and filtered. 24 ml of ethanol and 15.35 ml (90.6 mmol) of 6M HCl solution in isopropanol are added to the filtrate and the resulting mixture is refluxed for 4 hours then cooled to 0°C. The precipitate is filtered, washed with 16 ml of acetone and 16 ml of toluene and dried under vacuum to give 21.94 g of compound (1). HCl salt. Yield: 86%.
NMR and MS data are identical to those of the literature.

## Claims

1. A process for preparing compound (1), or an acid addition salt thereof, 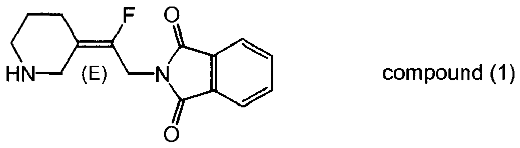 which is **characterized by** the steps of
a) reacting a compound of formula (I) with a compound of formula (II) in a reaction-inert solvent 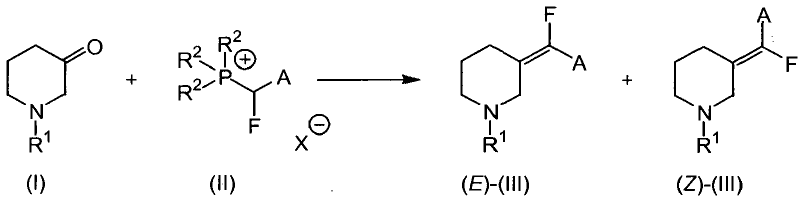 wherein
R¹ is hydrogen, C₁₋₄alkyl, aryl, arylmethyl, arylethyl, diphenylmethyl, allyl or 3-phenylallyl;
R² is C₁₋₆alkyl, phenyl or phenyl substituted with one substituent selected from halo, hydroxy, C₁₋₄alkyl, C₁₋₄alkoxy, trifluoromethyl, cyano or nitro;
A is C₁₋₄alkyloxycarbonyl, hydroxycarbonyl, aminocarbonyl, or cyano; and
X is halo;
wherein aryl is phenyl, or phenyl substituted with one or two C₁₋₄alkyloxy, halo, C₁₋₄alkyl, nitro, or di(C₁₋₄alkyl)amino,
b) reducing compound (III), which is a mixture of (*E*)-(III) and (*Z*)-(III), into compound (IV), which is a mixture of (*E*)-(IV) and (Z)-(IV), followed by isolating compound (*E*)-(IV) or a salt thereof as a precipitate. 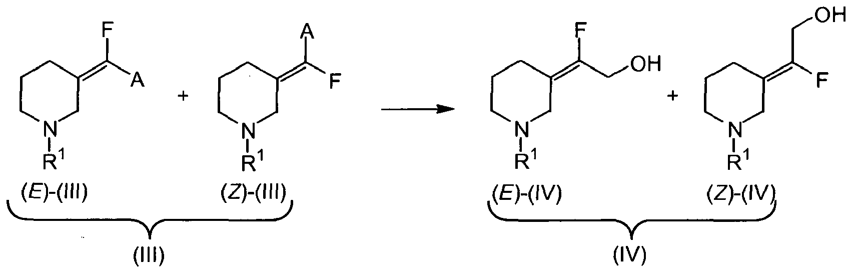 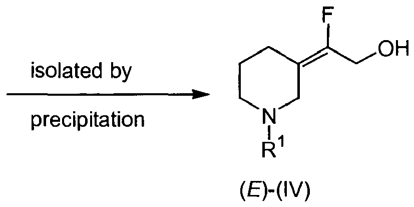
c) and, reacting a compound of formula (*E*)-(IV) with compound (V) under Mitsunobu reaction conditions thereby obtaining a compound of formula (VI) which is converted into compound (1), optionally in the form of an acid addition salt, by removing substituent R¹ using art-known deprotection methods.

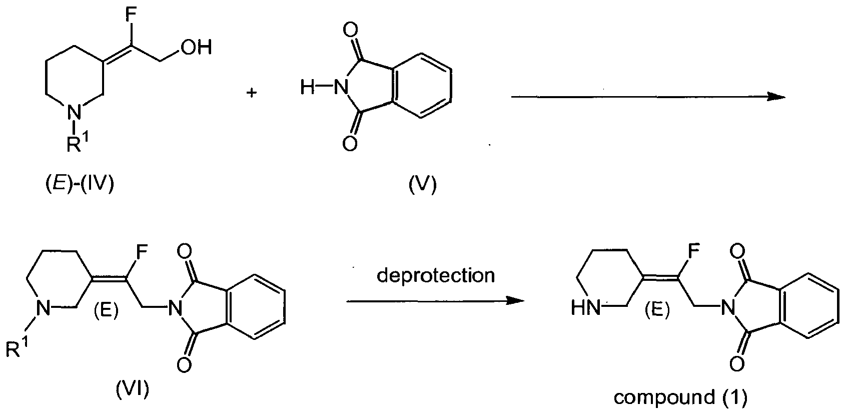

2. The process according to claim 1 wherein R¹ is C₁₋₄alkyl, aryl, arylmethyl, arylethyl, diphenylmethyl, allyl or 3-phenylallyl, wherein aryl is phenyl.

3. The process according to claim 2 wherein R² is methyl, ethyl, butyl, isobutyl or phenyl, X is bromo and A is hydroxycarbonyl, aminocarbonyl, cyano or C₁₋₄alkyloxycarbonyl wherein C₁₋₄alkyl is methyl, ethyl, or *tert*-butyl.

4. The process according to any one of claims 1 to 3 wherein R¹ is arylmethyl wherein aryl is phenyl, R² is methyl, X is bromo and A is C₁₋₄alkyloxycarbonyl wherein C₁₋₄alkyl is ethyl.

5. A process for preparing a compound of formula (III), which is **characterized by** the steps of reacting a compound of formula (I) with a compound of formula (II) in a reaction-inert solvent 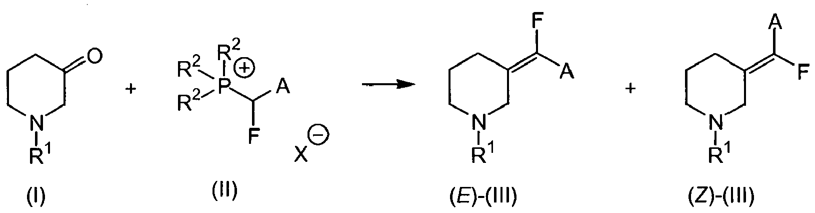 wherein
R¹ is hydrogen, C₁₋₄alkyl, aryl, arylmethyl, arylethyl, diphenylmethyl, allyl or 3-phenylallyl;
R² is C₁₋₆alkyl, phenyl or phenyl substituted with one substituent selected from halo, hydroxy, C₁₋₄alkyl, C₁₋₄alkoxy, trifluoromethyl, cyano or nitro;
A is C₁₋₄alkyloxycarbonyl, hydroxycarbonyl, aminocarbonyl, or cyano; and
X is halo;
wherein aryl is phenyl, or phenyl substituted with one or two C₁₋₄alkyloxy, halo, C₁₋₄alkyl, nitro, or di(C₁₋₄alkyl)amino,

6. The process according to claim 5 wherein R¹ is C₁₋₄alkyl, aryl, arylmethyl, arylethyl, diphenylmethyl, allyl or 3-phenylallyl, wherein aryl is phenyl.

7. The process according to claim 6 wherein R² is methyl, ethyl, butyl, isobutyl or phenyl, X is bromo and A is hydroxycarbonyl, aminocarbonyl, cyano or C₁₋₄alkyloxycarbonyl wherein C₁₋₄alkyl is methyl, ethyl, or *tert*-butyl.

8. The process according to claim 7 wherein R¹ is arylmethyl wherein aryl is phenyl, R² is methyl, X is bromo and A is C₁₋₄alkyloxycarbonyl wherein C₁₋₄alkyl is ethyl.

9. A process of reducing compound (III), which is a mixture of (*E*)-(III) and (*Z*)-(III), into compound (IV), which is a mixture of (*E*)-(IV) and (*Z*)-(IV), followed by isolating compound (*E*)-(IV) or a salt thereof as a precipitate. 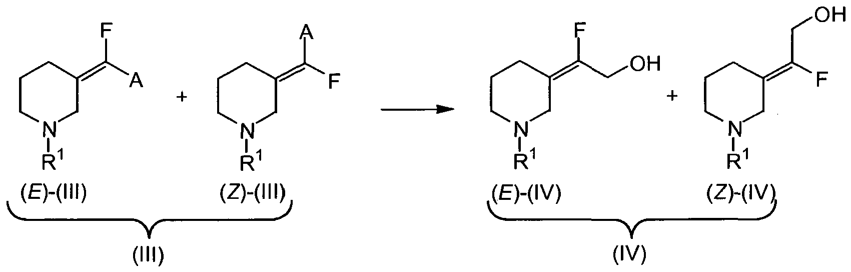 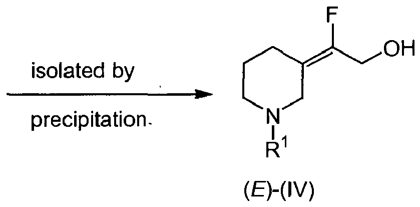 wherein
R¹ is hydrogen, C₁₋₄alkyl, aryl, arylmethyl, arylethyl, diphenylmethyl, allyl or 3-phenylallyl;
A is C₁₋₄alkyloxycarbonyl, hydroxycarbonyl, aminocarbonyl, or cyano; and
wherein aryl is phenyl, or phenyl substituted with one or two C₁₋₄alkyloxy, halo, C₁₋₄alkyl, nitro, or di(C₁₋₄alkyl)amino,

10. The process according to claim 9 wherein R¹ is C₁₋₄alkyl, aryl, arylmethyl, arylethyl, diphenylmethyl, allyl or 3-phenylallyl, wherein aryl is phenyl.

11. The process according to claim 9 wherein A is hydroxycarbonyl, aminocarbonyl, cyano or C₁₋₄alkyloxycarbonyl wherein C₁₋₄alky is methyl, ethyl, or tert-butyl.

12. The process according to claim 10 wherein R¹ is arylmethyl wherein aryl is phenyl, and A is C₁₋₄alkyloxycarbonyl wherein C₁₋₄alkyl is ethyl.

13. A process for reacting a compound of formula (*E*)-(IV) with compound (V) under Mitsunobu reaction conditions thereby obtaining a compound of formula (VI) which can be converted into compound (1), optionally in the form of an acid addition salt, by removing substituent R¹ using art-known deprotection methods, 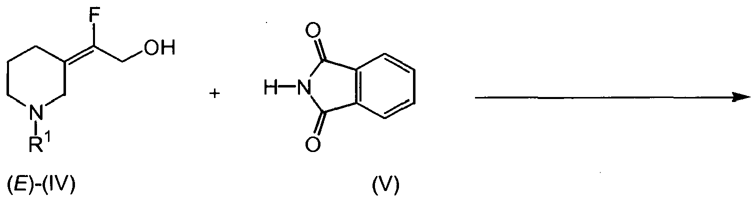 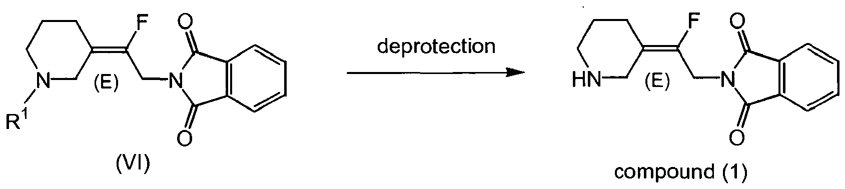 wherein
R¹ is hydrogen, C₁₋₄alkyl, aryl, arylmethyl, arylethyl, diphenylmethyl, allyl or 3-phenylallyl.

14. The process according to claim 13 wherein R¹ is arylmethyl wherein aryl is phenyl.

15. Compounds of formula (*E*)-(III) or (*E*)-(IV) 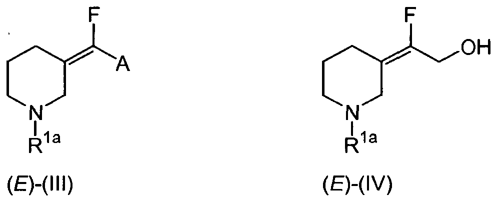 or acid addition salts thereof, wherein
R^{1a} is C₁₋₄alkyl, aryl, arylmethyl, arylethyl, diphenylmethyl, allyl or 3-phenylallyl;
A is C₁₋₄alkyloxycarbonyl, hydroxycarbonyl, aminocarbonyl, or cyano; and
wherein aryl is phenyl, or phenyl substituted with one or two C₁₋₄alkyloxy, halo, C₁₋₄alkyl, nitro, or di(C₁₋₄alkyl)amino.

16. Compounds of formula (*E*)-(III) or (*E*)-(IV) as claimed in claim 15 wherein R^{1a} is arylmethyl wherein aryl is phenyl.

17. Compound of formula (*E*)-(III) as claimed in claim 16 wherein A is C₁₋₄alkyloxycarbonyl.

18. Compound of formula (*E*)-(III) as claimed in claim 17 wherein A is C₁₋₄alkyloxycarbonyl wherein C₁₋₄alkyl is ethyl.

## Patentansprüche

1. Verfahren zur Herstellung von Verbindung (1) oder einem Säureadditionssalz davon, 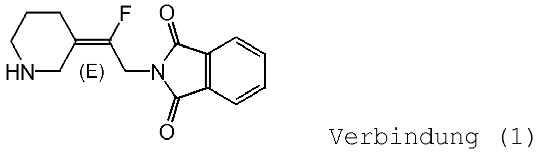 das **dadurch gekennzeichnet ist, dass** man:
a) eine Verbindung der Formel (I) in einem unter den Reaktionsbedingungen inerten Lösungsmittel mit einer Verbindung der Formel (II) umsetzt 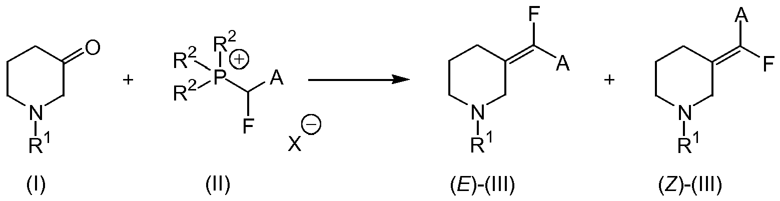 wobei
R¹ für Wasserstoff, C₁₋₄-Alkyl, Aryl, Arylmethyl, Arylethyl, Diphenylmethyl, Allyl oder 3-Phenylallyl steht;
R² für C₁₋₆-Alkyl, Phenyl oder Phenyl, das durch einen Substituenten, der aus Halogen, Hydroxy, C₁₋₄-Alkyl, C₁₋₄-Alkoxy, Trifluormethyl, Cyano oder Nitro ausgewählt ist, substituiert ist, steht;
A für C₁₋₄-Alkyloxycarbonyl, Hydroxycarbonyl, Aminocarbonyl oder Cyano steht und
X für Halogen steht;
wobei Aryl für Phenyl oder Phenyl, das durch ein oder zwei C₁₋₄-Alkyloxy, Halogen, C₁₋₄-Alkyl, Nitro oder Di(C₁₋₄-alkyl)amino substituiert ist, steht,
b) Verbindung (III), bei der es sich um ein Gemisch von (*E*) - (III) und (*Z*) - (III) handelt, zu Verbindung (IV), bei der es sich um ein Gemisch von (*E*) - (IV) und (*Z*) - (IV) handelt, reduziert und anschließend Verbindung (*E*)-(IV) oder ein Salz davon als Niederschlag isoliert 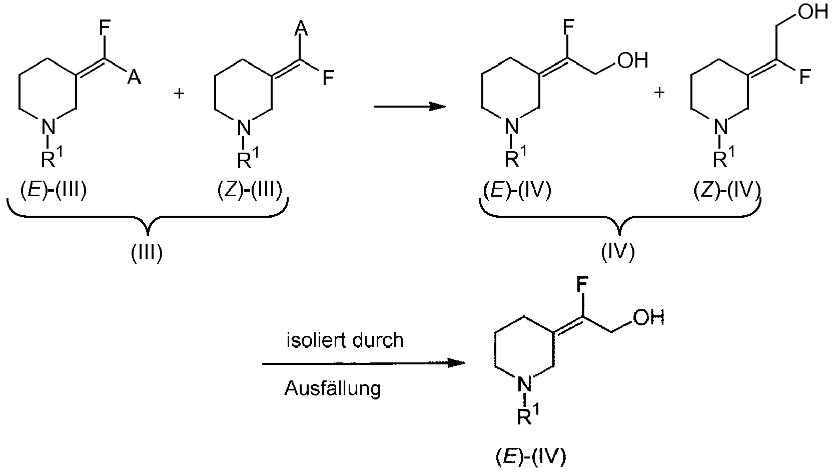
c) und eine Verbindung der Formel (*E*)-(IV) unter Mitsunobu-Reaktionsbedingungen mit Verbindung (V) umsetzt, wodurch man eine Verbindung der Formel (VI) erhält, die durch Entfernung des Substituenten R¹ nach in der Technik bekannten Entschützungsmethoden in Verbindung (1), gegebenenfalls in Form eines Säureadditionssalzes, umgewandelt wird.

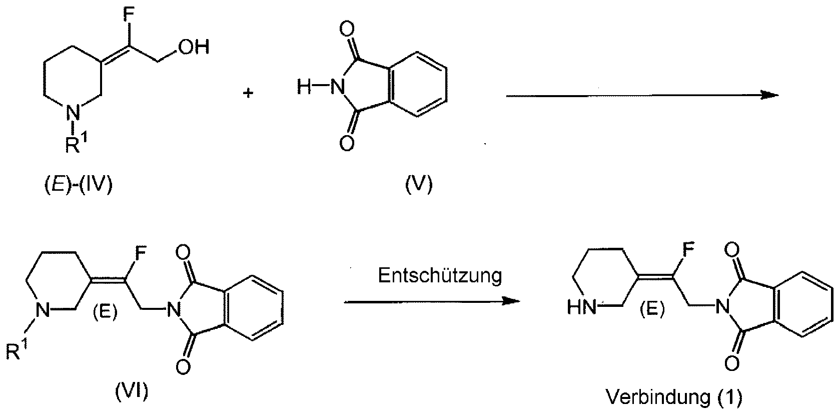

2. Verfahren nach Anspruch 1, wobei R¹ für C₁₋₄-Alkyl, Aryl, Arylmethyl, Arylethyl, Diphenylmethyl, Allyl oder 3-Phenylallyl steht, wobei Aryl für Phenyl steht.

3. Verfahren nach Anspruch 2, wobei R² für Methyl, Ethyl, Butyl, Isobutyl oder Phenyl steht, X für Brom steht und A für Hydroxycarbonyl, Amino-carbonyl, Cyano oder C₁₋₄-Alkyloxycarbonyl steht, wobei C₁₋₄-Alkyl für Methyl, Ethyl oder *tert*-Butyl steht.

4. Verfahren nach einem der Ansprüche 1 bis 3, wobei R¹ für Arylmethyl steht, wobei Aryl für Phenyl steht, R² für Methyl steht, X für Brom steht und A für C₁₋₄-Alkyloxycarbonyl steht, wobei C₁₋₄-Alkyl für Ethyl steht.

5. Verfahren zur Herstellung einer Verbindung der Formel (III), das **dadurch gekennzeichnet ist, dass** man eine Verbindung der Formel (I) mit einer Verbindung der Formel (II) in einem unter den Reaktionsbedingungen inerten Lösungsmittel umsetzt 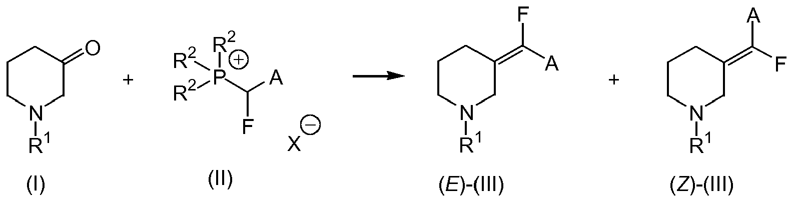 wobei
R¹ für Wasserstoff, C₁₋₄-Alkyl, Aryl, Arylmethyl, Arylethyl, Diphenylmethyl, Allyl oder 3-Phenylallyl steht;
R² für C₁₋₆-Alkyl, Phenyl oder Phenyl, das durch einen Substituenten, der aus Halogen, Hydroxy, C₁₋₄-Alkyl, C₁₋₄-Alkoxy, Trifluormethyl, Cyano oder Nitro ausgewählt ist, substituiert ist, steht;
A für C₁₋₄-Alkyloxycarbonyl, Hydroxycarbonyl, Aminocarbonyl oder Cyano steht und
X für Halogen steht;
wobei Aryl für Phenyl oder Phenyl, das durch ein oder zwei C₁₋₄-Alkyloxy, Halogen, C₁₋₄-Alkyl, Nitro oder Di(C₁₋₄-alkyl)amino substituiert ist, steht.

6. Verfahren nach Anspruch 5, wobei R¹ für C₁₋₄-Alkyl, Aryl, Arylmethyl, Arylethyl, Diphenylmethyl, Allyl oder 3-Phenylallyl steht, wobei Aryl für Phenyl steht.

7. Verfahren nach Anspruch 6, wobei R² für Methyl, Ethyl, Butyl, Isobutyl oder Phenyl steht, X für Brom steht und A für Hydroxycarbonyl, Amino-carbonyl, Cyano oder C₁₋₄-Alkyloxycarbonyl steht, wobei C₁₋₄-Alkyl für Methyl, Ethyl oder *tert*-Butyl steht.

8. Verfahren nach Anspruch 7, wobei R¹ für Arylmethyl steht, wobei Aryl für Phenyl steht, R² für Methyl steht, X für Brom steht und A für C₁₋₄-Alkyloxycarbonyl steht, wobei C₁₋₄-Alkyl für Ethyl steht.

9. Verfahren zur Reduktion von Verbindung (III), bei der es sich um ein Gemisch von (*E*) - (III) und (*Z*) - (III) handelt, zu Verbindung (IV), bei der es sich um ein Gemisch von (*E*) - (IV) und (*Z*) - (IV) handelt, und anschließenden Isolierung von Verbindung (E)-(IV) oder einem Salz davon als Niederschlag 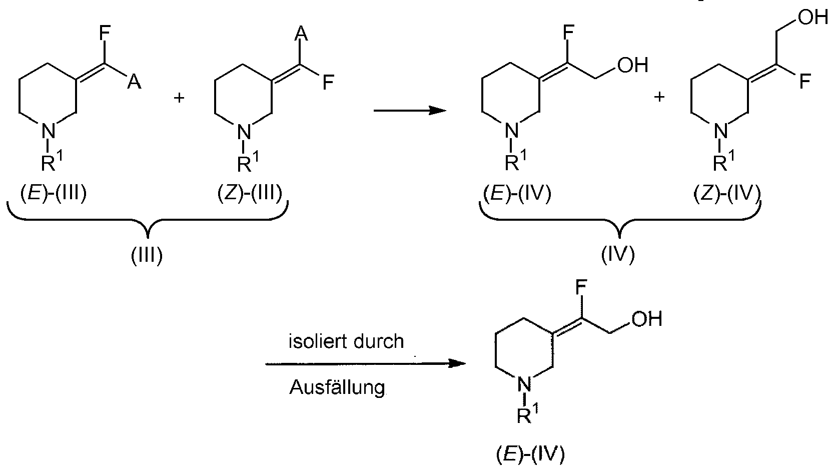 wobei
R¹ für Wasserstoff, C₁₋₄-Alkyl, Aryl, Arylmethyl, Arylethyl, Diphenylmethyl, Allyl oder 3-Phenylallyl steht;
A für C₁₋₄-Alkyloxycarbonyl, Hydroxycarbonyl, Aminocarbonyl oder Cyano steht und
wobei Aryl für Phenyl oder Phenyl, das durch ein oder zwei C₁₋₄-Alkyloxy, Halogen, C₁₋₄-Alkyl, Nitro oder Di(C₁₋₄-alkyl)amino substituiert ist, steht.

10. Verfahren nach Anspruch 9, wobei R¹ für C₁₋₄-Alkyl, Aryl, Arylmethyl, Arylethyl, Diphenylmethyl, Allyl oder 3-Phenylallyl steht, wobei Aryl für Phenyl steht.

11. Verfahren nach Anspruch 9, wobei A für Hydroxycarbonyl, Aminocarbonyl, Cyano oder C₁₋₄-Alkyloxycarbonyl steht, wobei C₁₋₄-Alkyl für Methyl, Ethyl oder *tert*-Butyl steht.

12. Verfahren nach Anspruch 10, wobei R¹ für Arylmethyl steht, wobei Aryl für Phenyl steht, und A für C₁₋₄-Alkoxycarbonyl steht, wobei C₁₋₄-Alkyl für Ethyl steht.

13. Verfahren zur Umsetzung einer Verbindung der Formel(*E*)-(IV) unter Mitsunobu-Reaktionsbedingungen mit Verbindung (V), wodurch man eine Verbindung der Formel (VI) erhält, die durch Entfernung des Substituenten R¹ nach in der Technik bekannten Entschützungsmethoden in Verbindung (1), gegebenenfalls in Form eines Säureadditionssalzes, umgewandelt werden kann, 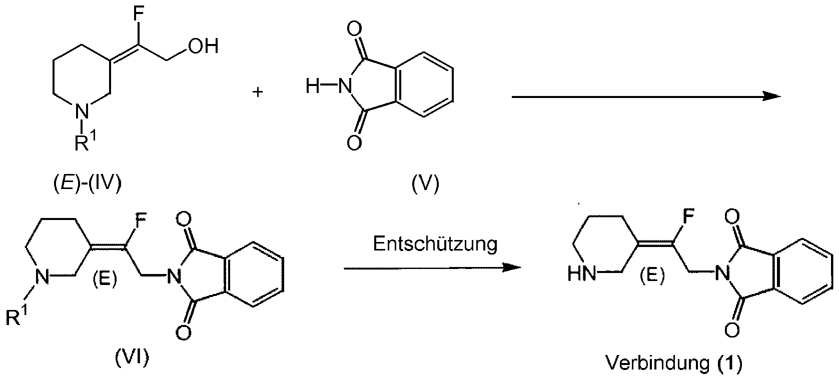 wobei
R¹ für Wasserstoff, C₁₋₄-Alkyl, Aryl, Arylmethyl, Arylethyl, Diphenylmethyl, Allyl oder 3-Phenylallyl steht.

14. Verfahren nach Anspruch 13, wobei R¹ für Arylmethyl steht, wobei Aryl für Phenyl steht.

15. Verbindungen der Formel (*E*) - (III) oder (*E*) - (IV) 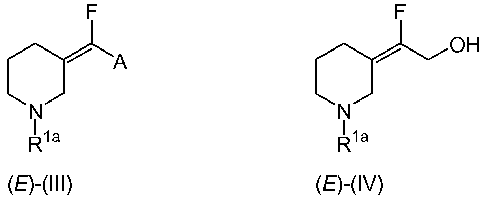 oder Säureadditionssalze davon, wobei
R^{1a} für C₁₋₄-Alkyl, Aryl, Arylmethyl, Arylethyl, Diphenylmethyl, Allyl oder 3-Phenylallyl steht;
A für C₁₋₄-Alkyloxycarbonyl, Hydroxycarbonyl, Aminocarbonyl oder Cyano steht und
wobei Aryl für Phenyl oder Phenyl, das durch ein oder zwei C₁₋₄-Alkyloxy, Halogen, C₁₋₄-Alkyl, Nitro oder Di(C₁₋₄-alkyl)amino substituiert ist, steht.

16. Verbindungen der Formel (*E*) - (III) oder (*E*) - (IV) nach Anspruch 15, wobei R^{1a} für Arylmethyl steht, wobei Aryl für Phenyl steht.

17. Verbindung der Formel (*E*)-(III) nach Anspruch 16, wobei A für C₁₋₄-Alkyloxycarbonyl steht.

18. Verbindung der Formel (*E*)-(III) nach Anspruch 17, wobei A für C₁₋₄-Alkyloxycarbonyl steht, wobei C₁₋₄-Alkyl für Ethyl steht.

## Revendications

1. Procédé pour la préparation de composé (1), ou d'un sel d'addition d'acide de celui-ci, 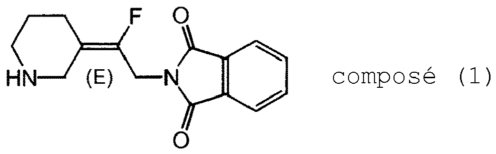 qui est **caractérisé par** les étapes consistant à
a) faire réagir un composé de formule (I) avec un composé de formule (II) dans un solvant inerte vis-à-vis de la réaction 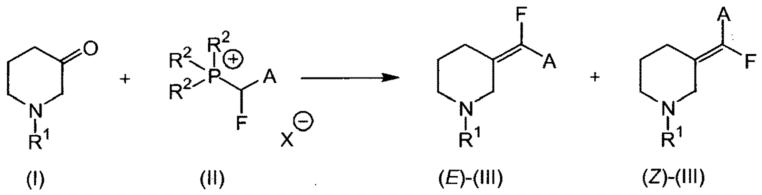
R¹ étant l'atome d'hydrogène ou un groupe alkyle en C₁₋₄, aryle, arylméthyle, aryléthyle, diphénylméthyle, allyle ou 3-phénylallyle ;
R² étant un groupe alkyle en C₁₋₆, phényle ou phényle substitué par un substituant choisi parmi les substituants halogéno, hydroxy, alkyle en C₁₋₄, alcoxy en C₁₋₄, trifluorométhyle, cyano ou nitro ;
A étant un groupe (alkyl en C₁₋₄) oxycarbonyle, hydroxycarbonyle, aminocarbonyle ou cyano ; et
X étant un groupe halogéno ;
le groupe aryle étant un groupe phényle ou phényle substitué par un ou deux substituants alkyloxy en C₁₋₄, halogéno, alkyle en C₁₋₄, nitro ou di (alkyl en C₁₋₄)amino,
b) réduire le composé (III), qui est un mélange de (*E*) - (III) et de (*Z*) - (III), en composé (IV), qui est un mélange de (*E*)-(IV) et de (*Z*) - (IV), et ensuite isoler le composé (*E*)-(IV) ou un sel de celui-ci sous forme d'un précipité, 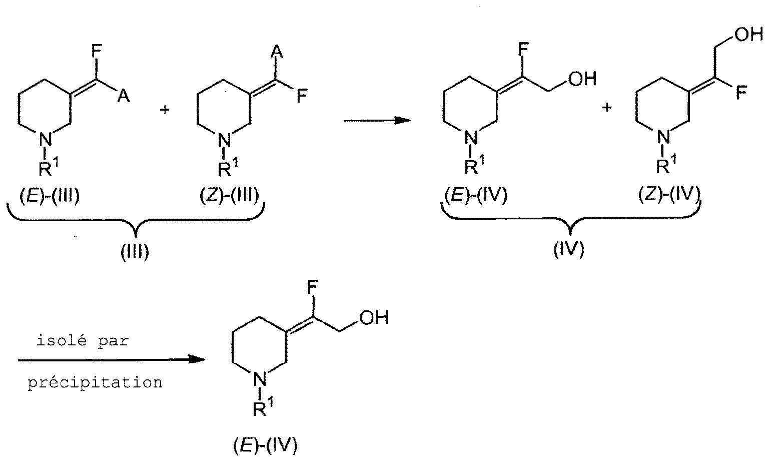
c) et faire réagir un composé de formule (*E*) - (IV) avec un composé (V) dans des conditions de réaction de Mitsunobu ce qui permet d'obtenir un composé de formule (VI) qui est converti en composé (1), facultativement sous la forme d'un sel d'addition d'acide, par élimination du substituant R¹ à l'aide de méthodes de déprotection connues dans l'art.

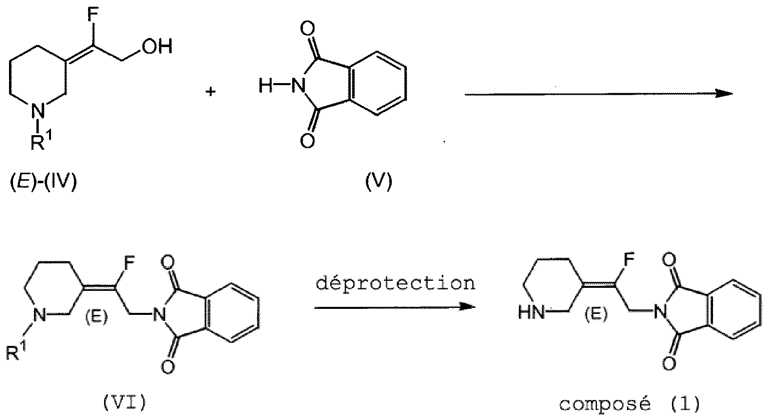

2. Procédé selon la revendication 1 dans lequel R¹ est un groupe alkyle en C₁₋₄, aryle, arylméthyle, aryléthyle, diphénylméthyle, allyle ou 3-phénylallyle, le groupe aryle étant le groupe phényle.

3. Procédé selon la revendication 2 dans lequel R² est le groupe méthyle, éthyle, butyle, isobutyle ou phényle, X est le groupe bromo et A est un groupe hydroxycarbonyle, aminocarbonyle, cyano ou (alkyl en C₁₋₄) oxycarbonyle dans lequel le groupe alkyle en C₁₋₄ est le groupe méthyle, éthyle ou *tert*-butyle.

4. Procédé selon l'une quelconque des revendications 1 à 3 dans lequel R¹ est un groupe arylméthyle dans lequel le groupe aryle est le groupe phényle, R² est le groupe méthyle, X est le groupe bromo et A est un groupe (alkyl en C₁₋₄)oxycarbonyle dans lequel le groupe alkyle en C₁₋₄ est le groupe éthyle.

5. Procédé pour la préparation d'un composé de formule (III), qui est **caractérisé par** les étapes consistant à faire réagir un composé de formule (I) avec un composé de formule (II) dans un solvant inerte vis-à-vis de la réaction 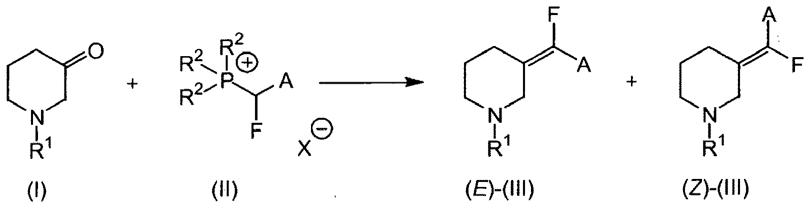 dans lequel
R¹ est l'atome d'hydrogène ou un groupe alkyle en C₁₋₄, aryle, arylméthyle, aryléthyle, diphénylméthyle, allyle ou 3-phénylallyle ;
R² est un groupe alkyle en C₁₋₆, phényle ou phényle substitué par un substituant choisi parmi les substituants halogéno, hydroxy, alkyle en C₁₋₄, alcoxy en C₁₋₄, trifluorométhyle, cyano ou nitro ;
A est un groupe (alkyl en C₁₋₄)oxycarbonyle, hydroxycarbonyle, aminocarbonyle ou cyano ; et
X est un groupe halogéno ;
le groupe aryle étant un groupe phényle ou phényle substitué par un ou deux substituants alkyloxy en C₁₋₄, halogéno, alkyle en C₁₋₄, nitro ou di (alkyl en C₁₋₄)amino.

6. Procédé selon la revendication 5 dans lequel R¹ est un groupe alkyle en C₁₋₄, aryle, arylméthyle, aryléthyle, diphénylméthyle, allyle ou 3-phénylallyle, le groupe aryle étant le groupe phényle.

7. Procédé selon la revendication 6 dans lequel R² est le groupe méthyle, éthyle, butyle, isobutyle ou phényle, X est le groupe bromo et A est un groupe hydroxycarbonyle, aminocarbonyle, cyano ou (alkyl en C₁₋₄)oxycarbonyle dans lequel le groupe alkyle en C₁₋₄ est le groupe méthyle, éthyle ou *tert*-butyle.

8. Procédé selon la revendication 7 dans lequel R¹ est un groupe arylméthyle dans lequel le groupe aryle est le groupe phényle, R² est le groupe méthyle, X est le groupe bromo et A est un groupe (alkyl en C₁₋₄)oxycarbonyle dans lequel le groupe alkyle en C₁₋₄ est le groupe éthyle.

9. Procédé de réduction de composé (III), qui est un mélange de (*E*) - (III) et de (*Z*) - (III), en composé (IV), qui est un mélange de (*E*)-(IV) et de (*Z*) - (IV), suivie de l'isolement de composé (*E*)-(IV) ou d'un sel de celui-ci sous forme d'un précipité 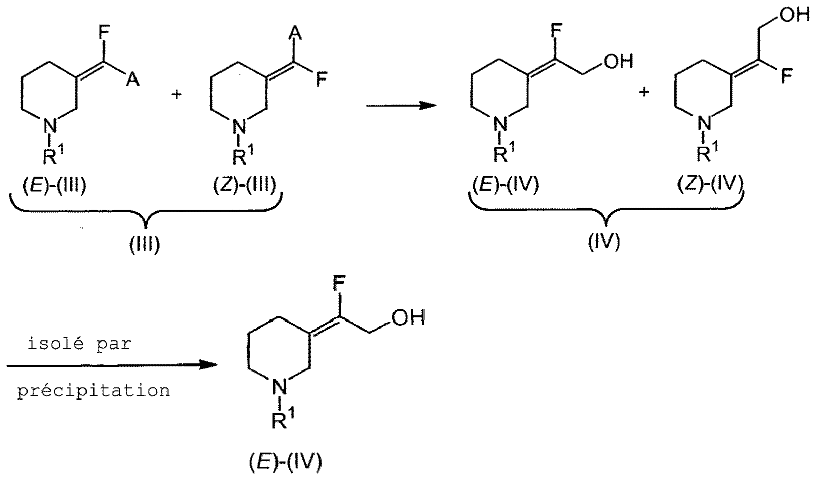 dans lequel
R¹ est l'atome d'hydrogène ou un groupe alkyle en C₁₋₄, aryle, arylméthyle, aryléthyle, diphénylméthyle, allyle ou 3-phénylallyle ;
A est un groupe (alkyl en C₁₋₄)oxycarbonyle, hydroxycarbonyle, aminocarbonyle ou cyano ; et
le groupe aryle étant un groupe phényle ou phényle substitué par un ou deux substituants alkyloxy en C₁₋₄, halogéno, alkyle en C₁₋₄, nitro ou di (alkyl en C₁₋₄)amino.

10. Procédé selon la revendication 9 dans lequel R¹ est un groupe alkyle en C₁₋₄, aryle, arylméthyle, aryléthyle, diphénylméthyle, allyle ou 3-phénylallyle, le groupe aryle étant le groupe phényle.

11. Procédé selon la revendication 9 dans lequel A est un groupe hydroxycarbonyle, aminocarbonyle, cyano ou (alkyl en C₁₋₄)oxycarbonyle dans lequel le groupe alkyle en C₁₋₄ est le groupe méthyle, éthyle ou *tert*-butyle.

12. Procédé selon la revendication 10 dans lequel R¹ est un groupe arylméthyle dans lequel le groupe aryle est le groupe phényle et A est un groupe (alkyl en C₁₋₄)oxycarbonyle dans lequel le groupe alkyle en C₁₋₄ est le groupe éthyle.

13. Procédé pour la réaction d'un composé de formule (*E*)-(IV) avec un composé (V) dans des conditions de réaction de Mitsunobu ce qui permet d'obtenir un composé de formule (VI) qui peut être converti en composé (1), facultativement sous la forme d'un sel d'addition d'acide, par élimination du substituant R¹ à l'aide de méthodes de déprotection connues dans l'art, 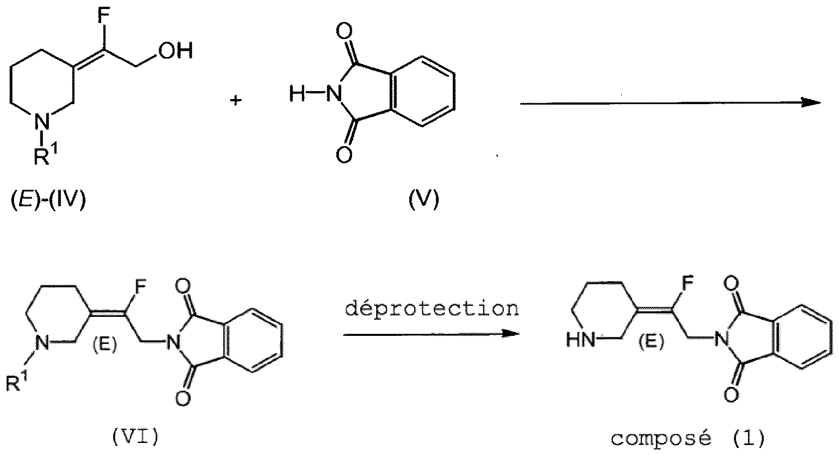 dans lequel
R¹ est l'atome d'hydrogène ou un groupe alkyle en C₁₋₄, aryle, arylméthyle, aryléthyle, diphénylméthyle, allyle ou 3-phénylallyle.

14. Procédé selon la revendication 13 dans lequel R¹ est un groupe arylméthyle dans lequel le groupe aryle est le groupe phényle.

15. Composés de formule (*E*) - (III) ou (*E*) - (IV) 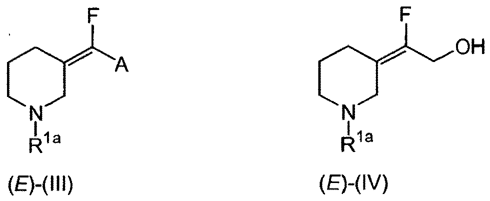 ou sels d'addition d'acide de ceux-ci, dans lesquels
R^{1a} est un groupe alkyle en C₁₋₄, aryle, arylméthyle, aryléthyle, diphénylméthyle, allyle ou 3-phénylallyle ;
A est un groupe (alkyl en C₁₋₄) oxycarbonyle, hydroxycarbonyle, aminocarbonyle ou cyano ; et
le groupe aryle étant un groupe phényle ou phényle substitué par un ou deux substituants alkyloxy en C₁₋₄, halogéno, alkyle en C₁₋₄, nitro ou di (alkyl en C₁₋₄) amino.

16. Composés de formule (*E*) - (III) ou (*E*) - (IV) selon la revendication 15 dans lesquels R^{1a} est un groupe arylméthyle dans lequel le groupe aryle est le groupe phényle.

17. Composé de formule (*E*) - (III) selon la revendication 16 dans lequel A est un groupe (alkyl en C₁₋₄)oxycarbonyle.

18. Composé de formule (*E*) - (III) selon la revendication 17 dans lequel A est un groupe (alkyl en C₁₋₄)oxycarbonyle dans lequel le groupe alkyle en C₁₋₄ est le groupe éthyle.
